# EUROPEAN PATENT SPECIFICATION

(11) **EP 2 382 226 B1**
(45) Date of publication and mention of the grant of the patent: **04.12.2013**
(21) Application number: 09806138.5
(22) Date of filing: 18.12.2009
(51) Int. Cl.: C07H 1/00, C07H 3/06

(54) **PROCESS FOR THE SYNTHESIS OF L-FUCOSYL DI- OR OLIGOSACCHARIDES AND NOVEL 2,3,4 TRIBENZYL-FUCOSYL DERIVATIVES INTERMEDIATES THEREOF**
VERFAHREN ZUR SYNTHESE VON L-FUCOSYL-DI- ODER OLIGOSACCHARIDEN UND NEUE 2,3,4-TRIBENZYLFUCOSYLDERIVATE ALS ZWISCHENPRODUKTE DAFÜR
PROCÉDÉ DE SYNTHÈSE DE DISACCHARIDES OU D'OLIGOSACCHARIDES DE L-FUCOSYLE ET DE NOUVEAUX INTERMÉDIAIRES DE CEUX-CI DU TYPE 2,3,4-TRIBENZYLFUCOSYLE

(30) Priority: 18.12.2008 IT FI20080244
(43) Date of publication of application: 02.11.2011
(73) Proprietor: Inalco S.p.A., 20139 Milan (IT)
(72) Inventor: SALSINI, Liana, I-55047 Seravezza (IT); MANONI, Marco, I-50059 Vinci (IT); CIPOLLETTI, Giovanni, I-20143 Milano (IT)
(74) Representative: Valenza, Silvia
(86) International application number: PCT/IB2009/055841
(87) International publication number: WO 2010/070616

(56) References cited:
- H. KUNZ, C. UNVERZAGT: "Synthesis of alpha-fucosyl glycosides using 4-methoxybenzyl (Mpm) protected fucosyl donors" J. PRAKT. CHEM., vol. 334, 1992, pages 579-583, XP002545666
- H. KUNZ, C. UNVERZAGT: "Schutzgruppenabhängige Stabilität von Intersaccharid-Bindungen - Synthese eines Fucosyl-Chitobiose-glycopeptids" ANGEW. CHEM., vol. 100, 1988, pages 1763-1765, XP002545667
- K. VON DEM BRUCH, H. KUNZ: "Synthese von N-Glycopeptid-Clustern mit LewisX-Antigen-Seitenketten und deren Bindung an Trägerproteine" ANGEW. CHEM., vol. 106, 1994, pages 87-89, XP002545668
- P. SÖDERMAN ET AL.: "Synthesis of trifucosylated N-linked hexasaccharide of a glycoprotein from Haemonchus contortus" EUR. J. ORG. CHEM., 2002, pages 1614-1618, XP002545669
- W. KINZY, A. LÖW: "Kohlenhydrat-Bausteine zum Aufbau Tumor-assoziierter Antigene. Synthese von Lewis Y-Determinanten" CARBOHYDRATE RES., vol. 245, 1993, pages 193-218, XP002545670
- S.A. ABBAS ET AL.: "Synthesis of O-alpha-L-fucopyranosyl-(1-2)-O-beta-D-gal actopyranosyl-(1-4)-D-glucopyranose (2'-O-alpha-L-fucopyranosyl-lactose)" CARBOHYDRATE RES., vol. 88, 1991, pages 51-60, XP002545671

## Description

### FIELD OF THE INVENTION

The present invention relates to the field of chemical synthesis of oligosaccharides and in particular the preparation of L-fucosyl di- or oligo-saccharides

### STATE OF THE ART

The human milk is rich of oligosaccharides. Up to today more then 130 different compounds have been isolated and identified.

Although in the past the nutritional importance of these compounds has been undervaluated, on the other hand it has been recently demonstrated their great importance for breast -fed newborns (L. Bode, J. Nutr. 2006, 136, 2127-2130; J. Kemsley, Chem. & Eng. News 2008, 86(39), 13-17); these oligosaccharides, besides further biological functions, inhibit the adhesion of bacteria to the epithelial cell surface, which has been demonstrated to be the main crucial moment for the beginning of a infective process. Fuc-α-(1-2)-Gal-β-(I-4)-Glc (2'-O-fucosyllactose, CAS N° 41263-94-9) e Gal-β-(1-4)-[Fuc-α-(I-3)]-Glc are the most abundant (Chaturvedi P. et al. Glycobiology. 2001. 11,5, 365-372) and it is not clear if the anti-adhesive properties of these fucosyl oligosaccharides are due to the presence of fucose (6-deoxy-L-galactose) or to the position of glycosilation. It is then necessary to have methods for the synthesis of these oligosaccharides to be able to study and understand better the biological processes in which they are involved as well as to add them into baby food as for example artificial milk, dietary supplements or pharmaceutical formulations.

Numerous synthetic procedures for the preparation of fucosyl oligosaccharides have been reported to the state of the art as for example:
- H.M. Flowers et al. Carbohydrate Research, 4, (1967),189-195;
- R.R. Schmidt and B. Wegman, Carbohyd. Res. (1988), 184, 254-261;
- S. A. Abbas et al, Carbohydr. Res., 88 (1981) 51-60;
- A. Fernandez-Mayorales et. al., Carbohydr. Res., 154 (1986) 93-101;
- R. K. Jain et al., Carbohydrate Research (1991), 212, C1-C3;
- M. Izumi, et a., J. Org. Chem. (1997), 62, 992-998;
- A. Rencurosi et al., J. Carbohydrate Chem. (2001), 20, 761-765;
- L. Panza et al., Carbohydrate Research 337 (2002), 1333-1342
- A. Rencurosi et al., Eur. J. Org. Chem. (2003), 1672-1680.
- W. Kinzy et al., Carbohyd. Res., 245 (1993), 193-218.

The already known synthesis present numerous protection and deprotection steps of the hydroxyl groups and all of them include many chromatographic purifications and then are difficult to be applied at industrial scale.

Otherwise the synthesis by enzymatic or fermentative way of fucosyl oligosaccharides like for example for fucosyl lactosides and fucosyl-acetyl lactosamine cannot allow to perform cost-effective process due to the high cost ot the enzymes, the use of not easily available substrates (GDP-fucose) and the low yields so far obtained (P. A. Prieto et al. US 5,945,314; S. Drouillard et al., Angew. Chem. Int. Ed. (2006), 45(11), 1778-1780; T. Murata et a. J. Carbohyd. Chem. (2003), 22(5), 309-316; E.Beat et al., Can. J. Chem., (2000), 78(6), 892-904).

It is then clear the need to develop processes which can allow to obtain fucosyl di-and oligosaccharides by a number of easy steps feasible at industrial scale which made use of easy isolations and purifications of the intermediates and the final product so not to compromise the yield and the purity.

### SUMMARY OF THE INVENTION

The present invention solves the above-mentioned problem by means of a process, feasible even at industrial scale, for the synthesis of fucosyl derivatives of formula (I) 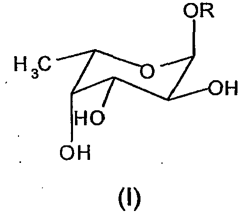 wherein
R is a monosaccharide, a disaccharide or an oligosaccharide with free hydroxy groups;
characterized in that it comprises the use of an intermediate of formula (IV) 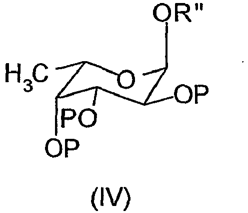 wherein P independently among each other are benzyl groups of formula 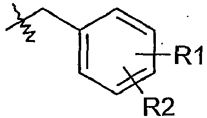 wherein R1 is chosen among chlorine, bromine, alkoxy and nitro; R2 is chosen among hydrogen, chlorine, bromine, alkoxy and nitro;
R" is, correspondingly to R, a monosaccharide, a disaccharide or an oligosaccharide whose hydroxy groups are free or partially protected by benzyl groups.

The compounds of formula (IV) as above described are surprisingly easy to isolate and purify by crystallization and as a consequence, they permit, even at industrial scale, easy purification and isolation of fucosyl derivatives of formula (I).

For an aspect object of the present invention are compounds 2,3,4-tri-O-benzyl-L-fucosyl derivatives of formula (II) or (IV) 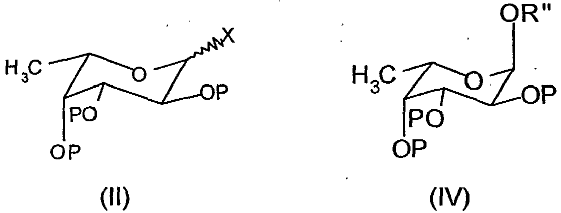 wherein
P independently among each other are benzyl groups of formula 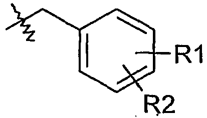 wherein R1 is chosen among chlorine, bromine, alkoxy and nitro; R2 is chosen among hydrogen, chlorine, bromine, alkoxy and nitro;
X is imidate;
R" is, correspondingly to R, a monosaccharide, a disaccharide or an oligosaccharide whose hydroxy groups are free or partially protected by benzyl groups.
proviso that for a compound of formula (II) when X is trichloroacetimidate and R1 is p-chloro, R2 is other than hydrogen.

Further object of the present invention is the use of compounds of formula (II) wherein
X is an activator of the anomeric Carbon chosen between an imidate or an halogen;
P independently among each other are benzyl groups of formula 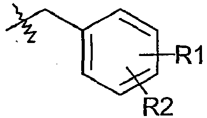 wherein R1 is chosen among chlorine, bromine, alkoxy and nitro; R2 is chosen among hydrogen, chlorine, bromine, alkoxy and nitro;
as fucosyl-donors for the synthesis of compounds of formula (IV) as above described.

Further object of the present invention is use of compounds of formula (II) as above described as fucosyl-donors for the synthesis of compounds of formula (I) as above described by means of the process of the present invention.

### DETAILED DESCRIPTION

The present invention relates to a process, feasible even at industrial level, for the synthesis of fucosyl derivatives of formula (I) 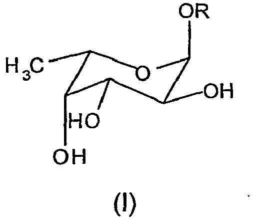 wherein
R is a monosaccharide, a disaccharide or an oligosaccharide with free hydroxy groups;
characterized in that it comprises the use of an intermediate of formula (IV) 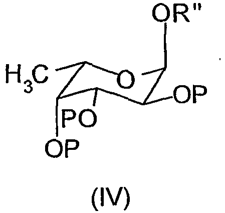 wherein P independently among each other are benzyl groups of formula 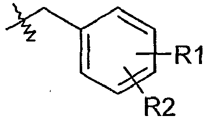 wherein R1 is chosen among chlorine, bromine, alkoxy and nitro; R2 is chosen among hydrogen, chlorine, bromine, alkoxy and nitro;
R" is, correspondingly to R, a monosaccharide, a disaccharide or an oligosaccharide whose hydroxy groups are free or partially protected by benzyl groups.

The compounds of formula (IV) as above described are surprisingly easy to isolate and purify by crystallization and as a consequence, they permit, even at industrial scale, easy purification and isolation of fucosyl derivatives of formula (I) In a preferred embodiment said process comprises a step c) for the removal of the intermediate (IV) of the benzyl groups to obtain a compound of formula (I).

For a more preferred embodiment said process comprises before step c) the following steps:
a) coupling of a donor 2,3,4-tri-O-benzyl-fucopyranosyl derivative of formula (II) 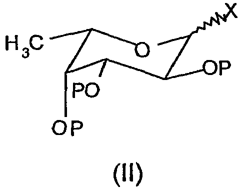 wherein P is as above described; X is an anomeric carbon activator;
   with a monosaccharide, disaccharide or oligosaccharide glycosyl acceptor of formula R'OH wherein R' is, correspondingly to R, a monosaccharide, disaccharide or oligosaccharide suitably protected whose hydroxy groups are at the most of 2 free, optionally are partially protected by benzyl groups, the remaining hydroxy groups are protected with protecting groups, known at state of the art, such to be removed in conditions such as to preserve benzyl groups and particularly to preserve groups -OP present on the fucosyl moiety;
   to obtain intermediates of formula (III) 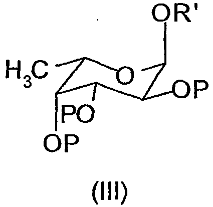 wherein P and R' are as above described;
b) removal of R' saccharide moiety protecting groups in conditions such as to preserve -OP groups present on the fucosyl moiety and optionally preserve benzyl groups present on R';
   to obtain intermediates of formula (IV) 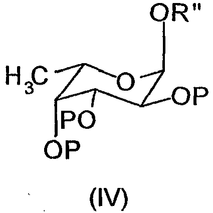 wherein R" and P are as above described.

The compounds of formula (IV) as above described are surprisingly easy to isolate and purify by crystallization, even after consecutive processing of the crude products of the steps a) and b); by consequence they allow, even at industrial scale, easy purification and isolation of fucosyl derivatives (I).

The crystallization of the intermediate (IV) is preferably performed directly from the reaction medium without the addition of further solvents or by the addition of an appropriate co-solvent for crystallization.

Hydroxy groups protected by benzyl groups means -OCH₂Ph or -OP groups.

Hydroxy groups partially protected by benzyl groups means that given an "n" number of hydroxy groups present on R' and R", the maximum number of them which are protected by benzyl groups is n/2 (when n is an even number) or (n-1)/2 (when n is an odd number). For example when R" is galactose then n=4 and then the term "partially protected" means that one or at the most two hydroxy groups are protected by benzyl groups while the remaining three or at the least two hydroxy groups are free; when R" is lactose then n=7 and then the term "partially protected" means that one , two or at the most three hydroxy groups are protected by benzyl groups while the remaining six, five or at the least four hydroxy groups are free.

Said R, R' and R" correspondingly deprotected , suitably protected or partially protected groups as above described are preferably chosen among lactose, fucose, (2-acetylamino)-lactose, (2-amino)-lactose, (2-azido)-lactose, Lacto-N-biose, galactose, glucose, (2-acetylamino)-glucose, (2-amino)-glucose and (2-azido)-glucose; more preferably are chosen among 2'-lactose, 3-lactose, 3-(2-acetylamino)-lactose, 3-(2-amino)-lactose, 3-(2-azido)-lactose, 4-Lacto-N-biose, 2-galactose, 3-glucose, 3-(2-acetylamino)-glucose, 3-(2-amino)-glucose, 3-(2-azido)-glucose 4-(2-acetylamino)-glucose, 4-(2-amino)-glucose e 4-(2-azido)-glucose. Said anomeric carbon activator is preferably chosen between imidate or halogen; more preferably X is α-bromine or trichloroacetimidate.

The coupling a) between a donor of formula (II) and the acceptor of formula R'OH can be performed by already known techniques

An anomeric carbon activator means a substituent (X) linked to the position 1 of the glycosyl donor that, in the presence of a suitable promoter, works as a good leaving group inducing the formation of the glycosyl bond; for example are included: halogens, tri-haloacetymidates, thyoglycosides, sulphoxydes and n-pentenyls.

Specific reaction conditions are necessary for each of these groups (see for example B.G. Davies, Recent Developments in Oligosaccharide Synthesis, J. Chem. Soc., Perkin Trans. I, (2000), 2137-2160).

The success of a coupling reaction between two sugars depends on the reactivity of the donor and acceptor, by the promoter, by substituents on both saccharide units and naturally by the reaction conditions used (solvent, temperature, adding modalities), as well as by the type of sugar used as glycosyl donor (I. Robina et al. Glycosylation Methods in Oligosaccharide Synthesis. Part 1, Current Organic Synthesis (2008), vol. 5(1), 33-60, I. Robina et al. Glycosylation Methods in Oligosaccharide Synthesis. Part 2, Current Organic Synthesis (2008), vol. 5(2), 81-116).

Moreover, the coupling reaction can give a different configuration of the new glycosyl bond (formation of 1,2-cis, 1,2 trans bonds or mixture of the two ones) The main factor affecting the configuration is the nature of the protective groups present on the position 2 of the glycosyl donor (partecipating groups/non-partecipating groups, groups with or without anchimeric assistance etc).

By appropriate selection of the above-mentioned parameters , it is possible to obtain glycosides with α (1,2-cis) configuration.

For example when X is an imidate, in particular when X is trichloroacetimidate, the coupling can be suitably performed by using the "inverse procedure" as described in R. R. Schmidt and A. Toepfer (J. Carbohyd. Chem, 1993, 12(7), 809-822) using an aprotic solvent, as for example dichloromethane and diethyl ether, in presence of a Lewis acid such asBoron Trifluoride etherated or trimetilsilyl trifluoromethansulphonate, preferably trimetilsilyl trifluoromethansulphonate. When X is an halogen, and in particular when X is α-bromine, the coupling can be performed by the Koenigs-Knoor reaction by using as a promoter Ag(I) (Koenigs, W.; Knoor, E. Chem. Ber., 1901, 34, 957) or by using the Helferich modification which uses Hg(II) as a promoter (Helferich, B.; Zirner, J. Chem. Ber., 1962, 95, 2604).

The deprotection step b) of the saccharide moiety R' can be performed by using state of the art methods (T.W. Green and P.G.M. Wuts. Green's Protective Groups in Organic Synthesis. Ed. Wiley ed 4. 2006) which preserve unmodified the benzyl groups on the fucose and if necessary the benzyl groups on the saccharide moiety. Therefore the acceptor R'OH is preferably protected with groups which are removable by basic reaction condition or by weak acid hydrolysis as for example acyl and acetal groups.

The debenzylation step c) can be performed by state of the art methods, for example by a catalitic hydrogenation (W. Kinzy et al., Carbohyd. Res., 245 (1993), 193-218) or acid hydrolysis (M. Izumi, et a., J. Org. Chem. (1997), 62, 992-998, L. Panza et al., Carbohydrate Research 337 (2002), 1333-1342) preferably by catalytic hydrogenation.

Fucosyl donors of formula (II) as above described can be synthesized starting from L-fucose according to state of the art methods.For example for the preparation of a compound of formula (II) where X is trichloroacetimidate or bromine, it is possible to pass, as described in the scheme 1, through the preparation of the methyl fucoside and its 2,3,4-O-tri-benzyl derivatization with a benzyl bromide or chloride of formula P-Br or P-Cl, preferably P-Cl, wherein P is as above described.For the activation of the anomeric carbon as a trichloroacetimidate, the synthesis can continue with the acid hydrolysis of the methylfucoside and the formation of the imidate with trichloroacetonitrile and DBU (W. Kinzy et al., Carbohyd. Res., 245 (1993), 193-218).

For the activation of the anomeric carbon as an halide, preferably α-bromine, after the acid hydrolysis of the tribenzyl methylfucoside the synthesis can be continued with two consecutive steps to obtain the halide(Flowers et al. Carbohyd. Res. 1971, 18, 215-226).

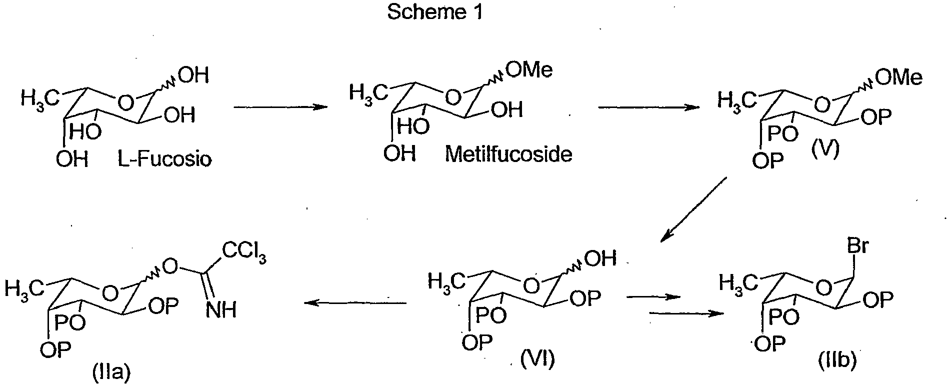

For a particularly preferred embodiment the present invention relates to a process for the synthesis of 2'-O-fucosyllactose, that is a compound of formula (I) wherein R is 2'-lactose, obtained by the above described process, according to what described in the scheme 2, and comprising the following steps:
a) coupling between a glycosyl donor of formula (II) as above described and a glycosyl acceptor 6'-O-substituted -2,3:5,6:3',4'-tri-O-isopropyliden-lactose dimethyl acetal of formula (VII) 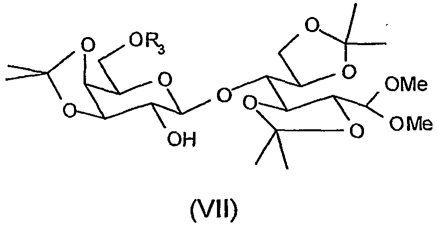 wherein R₃ is a group chosen among alkyl or aryl acyl, benzyl, P, trityl, silyl derivatives: preferably R₃ is acyl and more preferably acetyl or benzoyl optionally mono- or di- substituted by chlorine, bromine, alkoxy or nitro;
   to obtain the intermediate (IIIa) 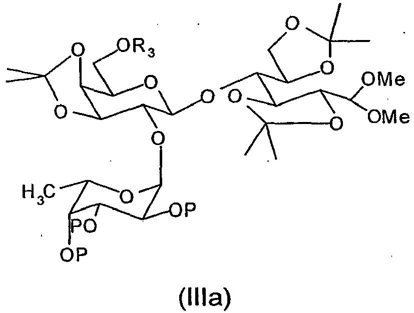 wherein P, and R3 are as above described;
b) deprotection comprising, as described in the scheme 3, the sequential steps of deprotection (b') and de-acetonation (b"); the deprotection (b') and the de-acetonation (b") may be performed without distinction in any order with the consequent passage through intermediates of formula (IIIb') or (IIIb"). 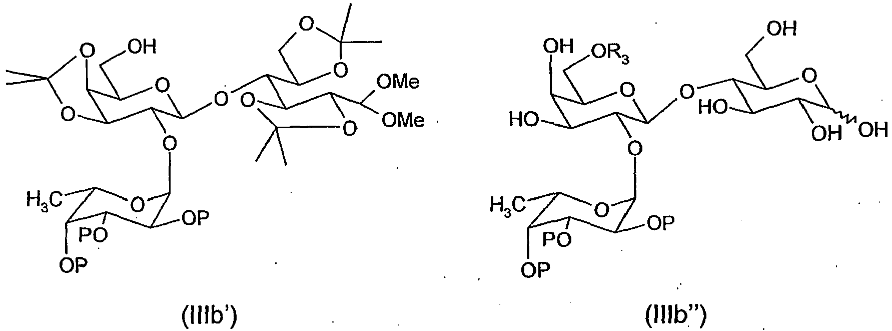 wherein P, and R₃ are as above described;
   to obtain the intermediates of formula (IV) wherein R" is 2'-lactose that is the formula (IVa) 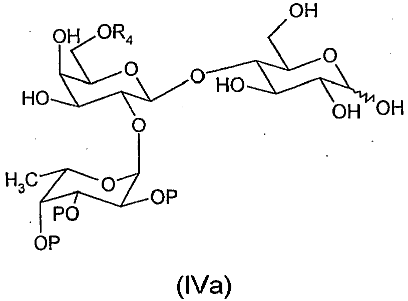 wherein P is as above described; R4 is hydrogen, benzyl or P;
c) debenzylation of the intermediate (IVa), through methodologies known at state of the art preferably by catalitic hydrogenation.

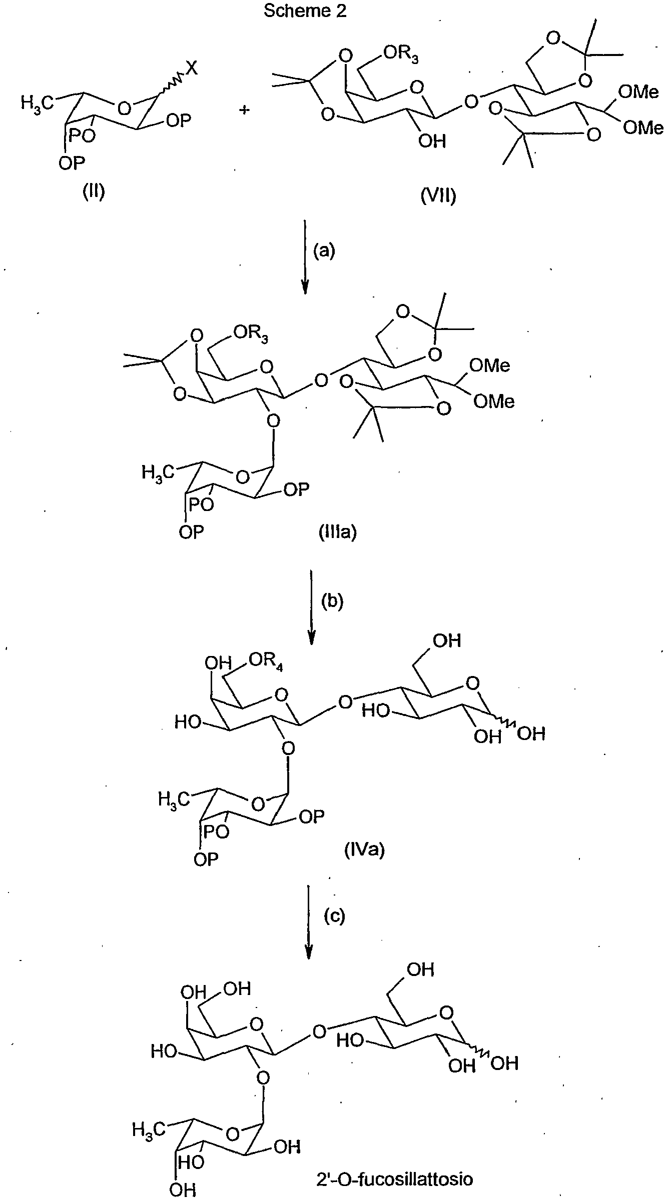

The deprotection b) is preferably accomplished passing through the intermediate (IIIb') when R3 is different from benzyl or P; in the case R3 is benzyl or P it is preferable to pass through the intermediate (IIIb") that in this case corresponds to the intermediate (IVa) and therefore it allows to save one step.

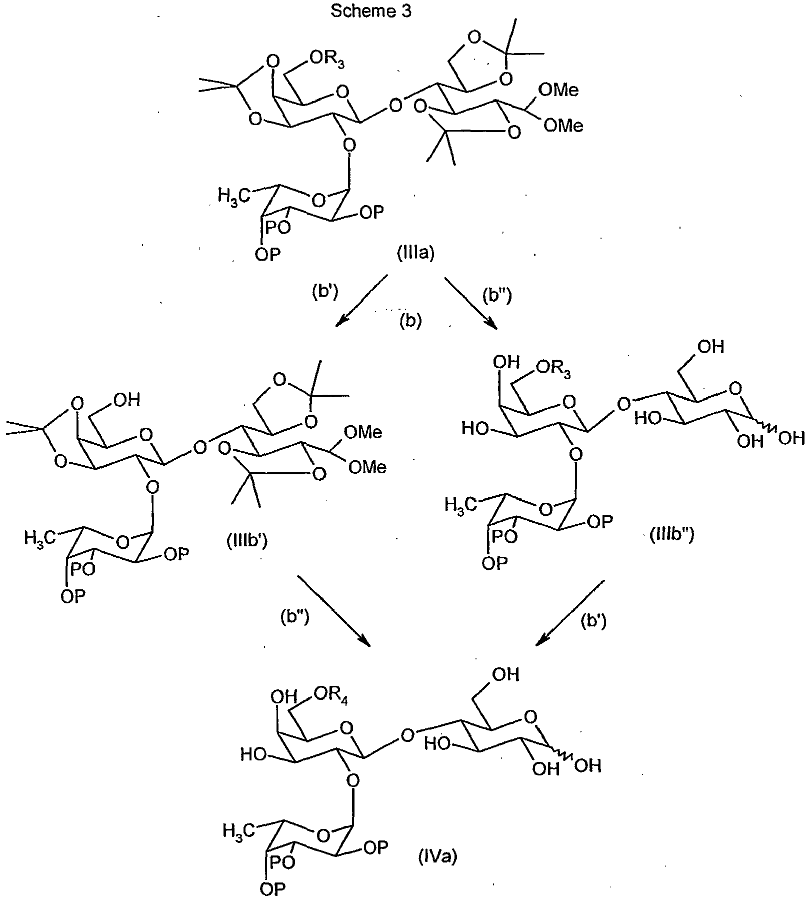

Said sequential deprotection phase (b) (Scheme 3) is preferably performed without the isolation of the intermediates (IIIb') and (IIIb") as above described which are sequentially used as crude products coming from the reactions (b') and (b"). When R₃ is acyl, the deprotection (b') (Scheme 3) is performed according to the procedures known at state of the art, preferably by using a base such as sodium methoxide, sodium ethoxide or sodium hydroxide, more preferably sodium hydroxide, and by using a primary alcohol as a solvent, preferably methanol or ethanol, more preferably methanol.

The de-acetonation (b") (Scheme 3) is achieved according to methods known at the state of the art, in presence of an acid, preferably HCl, in a polar solvent such as dimethylformamide, acetonitrile, water or a mixture of them; preferably mixtures of acetonitrile/water; more preferably a mixture of acetonitrile/water in the ratio comprised between 0.5:1 and 10:1; even more preferably between 1:1 and 8:1. The intermediates of formula (IVa) have just turned out to be surprisinglyeasy to crystallize even after they have been obtained by the processing of the crude reaction products of steps a) and b), in particular they may directly crystallize from the reaction medium without the addition of co-solvents.

The derivatives (IVa) as above described, unexpectedly compared to the known compound of formula (IVa) wherein P is a non-substituted benzyl (S. A. Abbas et al, Carbohydr. Res., 88 (1981) 51-60), turned out to be easy to isolate by crystallization even when obtained after the sequential processing of the intermediates (II), (IIIa), (IIIb') o (II), (IIIa), (IIIb") used as crude products respectively directly obtained from the steps a) and b), in particular with regard to the phase b) following the sequential deprotections (b') e (b"), or *vice versa*, achieved without the isolation of the intermediates (IIIb') e (IIIb") that are used as reaction crude products.

In particular, for the synthesis of 2'-O-fucosyl-lactose, when the above described process is performed by a donor of formula (II) wherein X=OC(NH)CCl3, P=p-chlorobenzyl or 2-chlorobenzyl with an acceptor of formula (VII) wherein R3 is benzoyl used as crude product, the compounds of formula (IVa) obtained through intermediates of formula (IIIb'), even though they are obtained after five chemical transformations performed by using crude intermediates, even more surprisingly they may directly crystallize from the de-acetonation (b") reaction medium without the addition of co-solvents.

The really unexpected result is that the intermediates of formula (IVa) are easy to crystallize from a reaction medium wherein they are contained in a concentration lower than 50% in weight while the remaining content is made of by-products and residues coming from the 5 previous chemical reactions.

The acceptor (VII) has been synthesized starting from monohydrate lactose by acetonation (Barili et al. Carbohyd. Res. 1997, 298, 75-84) and following selective protection of the 6' position.

The substituent R₃ on 6'position is preferably an acyl group, for example an acetyl group or a benzoyl or a benzoyl mono- or di- substituted with chlorine, bromine, alkoxy or nitro, preferably benzoyl (S. A. Abbas et al, Carbohydr. Res., 88 (1981) 51-60) as described in the scheme 4.

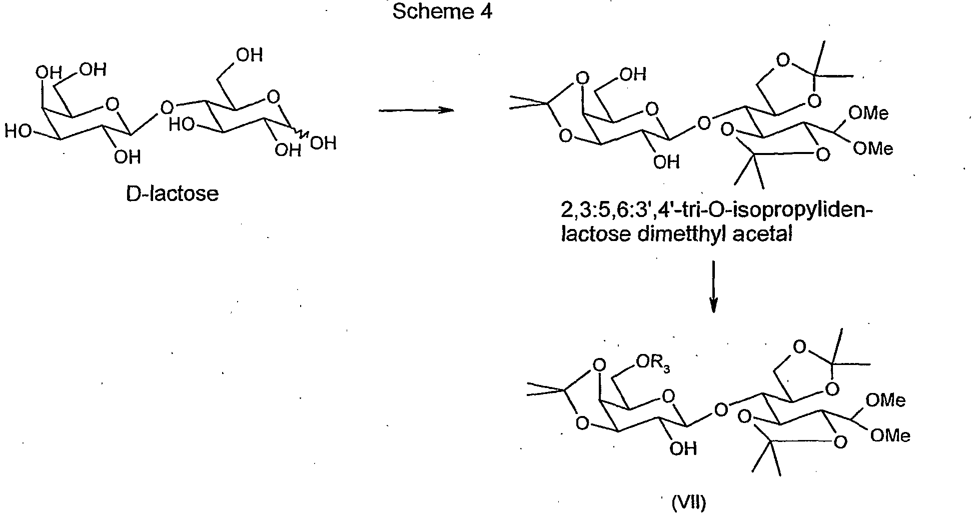

For an aspect, object of the present invention are compounds 2,3,4-tri-O-benzyl-L-fucosyl derivatives of formula (II) or (IV) 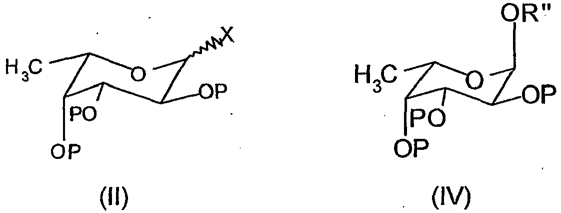 wherein
P independently among each other are benzyl groups of formula 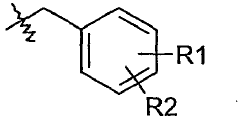 wherein R1 is chosen among chlorine, bromine, alkoxy and nitro; R2 is chosen among hydrogen, chlorine, Bromine, alkoxy and nitro;
X is imidate;
R" is a monosaccharide, a disaccharide or an oligosaccharide which hydroxy groups are free or partially protected by benzyl groups;
excluding that for a compound of formula (II) R2 is hydrogen when X is trichloroacetimidate and R1 is p-chloro,.
R2 is preferably hydrogen.
R1 is preferably chlorine.

In the case of compounds of formula (IV) R" is preferably chosen among lactose, fucose, (2-acetylamino)-lactose, (2-amino)-lactose, (2-azido)-lactose, Lacto-N-biose, galactose, glucose, (2-acetylamino)-glucose, (2-amino)-glucose and (2-azido)-glucose; which groups are free or partially protected by benzyl groups; more preferably are chosen among 2'-lactose, 3-lactose, 3-(2-acetilamino)-lactose, 3-(2-amino)-lactose, 3-(2-azido)-lactose, 4-Lacto-N-biose, 2-galactose, 3-glucose, 3-(2-acetylamino)-glucose, 3-(2-amino)-glucose, 3-(2-azido)-glucose 4-(2-acetylamino)-glucose, 4-(2-amino)-glucose e 4-(2-azido)-glucose which hydroxy groups are free or partially protected by benzyl groups.

In a particularly preferred embodiment the present invention provides compounds of formula (IV) wherein R" is 2'-lactose that is compounds of formula (IVa) 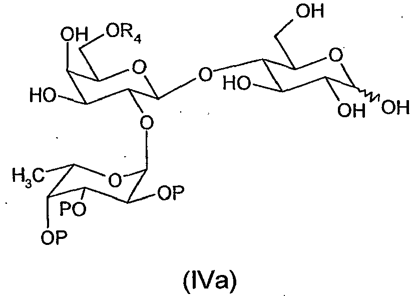 wherein P is as above described; R4 is hydrogen, benzyl or P;

In the case of compounds of formula (II) X is preferably trichloroacetimidate. The compounds of formula (IV) as above described are obtainable by means of the steps a) an b) of the above described process.

Moreover the present invention relates to the use of compounds of formula (II) wherein X is an anomeric-carbon activator chosen between an imidate or an halogen; preferably trichloroacetimidate or α-bromine;

P independently among each other are benzyl groups of formula 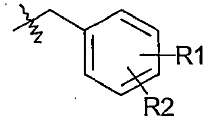 wherein R1 is chosen among chlorine, bromine , alkoxy and nitro; R2 is chosen among hydrogen, chlorine, bromine , alkoxy and nitro; preferably R2 is hydrogen and R1 is chlorine;
as fucosyl-donors for the synthesis of compounds of formula (IV) as above described.

Moreover the present invention relates to the use of compounds of formula (II) as above described as fucosyl-donors for the synthesis of compounds of formula (I) as above described by the process of the present invention.

Moreover the present invention relates to the use of compounds of formula (II) wherein
X is an anomeric activator chosen between an imidate or an halogen; preferably trichloroacetimidate or bromine;
P independently among each other are benzyl groups of formula 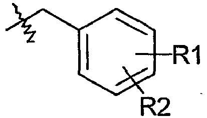 wherein R1 is chosen among chlorine, bromine , alkoxy and nitro; R2 is chosen among hydrogen, chlorine, bromine, methoxy, alkoxy and nitro; preferably R2 is hydrogen; more preferably R2 is hydrogen and R1 is p-chloro;
as fucosyl-donors for the synthesis of compounds of formula (IV) as above described.

Moreover the present invention relates to the use of compounds of formula (II) as above described as fucosyl-donors for the synthesis of compounds of formula (I) as above described by the process of the present invention.

According to the invention alkoxy means for example -OMe, -OEt, -O*n*Pr, -O*i*Pr,-O*n*Bu, -O*i*Bu, -O*t*Bu.

According to the invention halogen means fluorine, chlorine, bromine, iodine.

According to the invention alkyl is a linear or branched alkyl chain containing from 1 to 10 Carbon atoms optionally substituted by one or more groups chosen among halogen, hydroxy, alkoxy, nitro.

According to the invention aryl is benzene optionally substituted by one or more groups chosen from halogen, alkoxy, nitro.

According to the invention acyl means a group -OCO-alkyl , or -OCO-aryl wherein alkyl and aryl are as above defined.

According to the invention imidate means a group -OC(NH)-alkyl wherein alkyl is as above defined.

According to the invention trialkyl silyl means a chemical group made by a silicon atom linked to a 3 alkyl or aryl as substituents (the same or different) where alkyl and aryl are as above defined (for example tert-butyldimethylsilyl, tert-butyldiphenylsilyl or triisopropylsilyl).

According to the invention a monosaccharide is referred to a polyoxyaldheide (aldose) or a polyoxyketone (ketose) that is a simple sugar of formula (CH₂O)ₙ, CₙH₂ₙOₙ₋₁, CₙH₂ₙOₙ₋₁NH₂, CₙH₂ₙOₙ₋₁N₃ or CₙH₂ₙOₙ₋₁NHAc with n=3,4,5,6,7;

The definition comprises all the possible stereoisomers and all the open or cyclic forms, that is intramolecular semi acetals and semi-ketals as for example the pyranosidic and furanosidic forms; for example are comprised into the definition glyceraldheide, allose, altrose, arabinose, eritrose, fucose, galactose, glucose, glucosamine, N-acetyl-glucosamine, idose, lixose, mannose, psicose, ribose, deoxirobose, sorbose, tagatose, treose, xilose and the correspondent chetoses According to the invention a disaccharide is referred to a polyhydroxylate compound made by two monosaccharides linked through an acetalic bond that is both O-glycosyl and N-glycosyl bonds; the definition comprises all the possible stereoisomers and all the open or cyclic forms ; examples include lactose, lactosamine, N-acetyl-lactosamine, maltose, cellobiose, saccharose, trealose turanose.

According to the invention an oligosaccharide means a polymer having from three to 6 monosaccharides linked among each other by glycosyl bonds in such a manner to form linear or branch saccharide chains; are then included for example raffinose, melezitose, maltotriose, acarbose, stachiose.

The present invention may be better understood on the basis of the following experimental examples.

### EXPERIMENTAL SECTION

### Example 1

### Preparation of 2,3:5,6:3',4'-Tri-O-Isopropylidenelactose Dimetylacetal (1).

100.0 grams (278 mmoles) of lactose and 4.0 grams (21 mmoles) of p-toluen sulphonic acid have been suspended in 600 ml of 2,2 dimethoxy propane and the mixture has been heated to reflux.

After 90' the solution has been neutralized with triethylamine (3,2 ml, 2,3 mmoles), cooled at room temperature and concentrated under vacuum to obtain a thick syrup.

The syrup has been treated twice with toluene (2x 100ml) and again concentrated to dryness to remove the excess of base.

The residue has been dissolved in 10 volumes of a 9:1 methanol/water mixture and heated at reflux until the disappearance of the upper R_{f} spot (0,7 in ethyl acetate) was observed.

The solvent has been removed by distillation under vacuum and the final syrup has been dissolved in 300 ml of dichloromethane.

The organic layer has been washed twice with water and then dried on sodium sulphate, filtered and concentrated again under vacuum up to a syrup.

The crystallization of the residual from an ethyl acetate/hexane mixture gave 91,2 grams (179 mmoles, 64%) of 2,3:5,6:3',4'-tri-O-Isopropylidenelactose dimetylacetal (1).

¹³C NMR (75 MHz, CDCl₃): 110.47, 109.94, 108,38 (3 quaternary carbons of (CH₃)₂C=) groups; 107.20 (C1), 103.52 (C1'); 79.54, 78,24, 77.63, 75.88, 75.44, 74.75 74.18, 73.64; 64.57 (C6), 62.45 (C6'); 57.56 e 54.41 (2 OCH₃); 28.17, 27.10, 26.30, 26.30, 25.72, 24.01 (6 CH₃C=).

### Example 2

### Preparation of 6'-O-Benzoyl-2,3:5,6:3',4'-Tri-O-Isopropylidenelactose Dimethylacetal (2).

### (compound of the formula VII with R₃= benzoyl)

51.0 grams (100 mmoles) of 1 and 17.5 ml (126 mmoles) of triethylamine have been dissolved in 510 ml of dichloromethane.

A solution of 14,6 ml (126 mmoles) of benzoyl chloride in 245 ml of dichloromethane has been added drop-wise over a period of 30' to the previously prepared solution cooled at 0°C.

After 3 hours at 0°C the solution was warmed up to room temperature and washed with a saturated solution of sodium bicarbonate, then with water.

After separation , the organic layer has been dried over sodium sulphate, filtered and concentrated up to a syrup (73.5 g) of crude 6'-O-benzoyl-2,3:5,6:3',4'-tri-O-isopropylidenelactose dimetylacetal which has been used in the coupling reactions without further purification.

¹³C NMR (75 MHz, CDCl₃): 166.35 (ArCOO); 133.18, 130.44, 129.79, 128.44 (C Ar); 110.39, 110.14, 108.31 (3 C quat. Isopropylidene groups); 105.06 (C1), 103.81 (C1'); 79.07, 77.86, 77.82, 76.46, 75.21, 74.29, 73.45, 71.63; 64.74 (C6), 64.01(C6'); 56.34, 53.24 (2 OCH₃); 28.13, 27.23, 26.43, 26.30, 25.72, 24.60 (6 CH₃C=).

### Example 3

### Preparation of Methyl 6-Deoxy-L-Galactopyranoside (Methyl L-fucopyranoside) (3)

200 grams (1.22 moli) of L-fucose and 200 grams of Amberlite IR120, H⁺ form, have been suspended in 2 liters of methanol and heated by reflux under vigorous stirring for 24 hours.

After cooling at room temperature the resin has been filtered and the solution has been evaporated under vacuum.
145.4 grams (816 mmoles, 67%) of methyl 6-deoxy-L-galactopyranoside (3) have been obtained after crystallization of the residue from ethyl acetate

¹³C NMR (75 MHz, D₂O), α-anomer: 100.1(C1), 72.4, 70.2, 68.5, 67.1, 15.9 (C6), 55.7 (OCH₃); β-anomer: 104.4 (C1), 73.6, 72.0, 71.5, 71.1,15.9 (C6), 57.8 (OCH₃).

After filtration of the crystals, the mother liquors, once concentrated up to syrup, can be reprocessed as described above to obtain additional 43.0 g (20%) of compound 3 with the same characteristics of the previous one. Total yield 87%.

### Example 4

### Preparation of Methyl 2,3,4-Tri-O-(4-Chlorobenzyl)-6-Deoxy-L-Galactopyranoside (4).

### (compound of formula V with P= 4-chlorobenzyl)

10.0 grams (56 mmoles) of the 3 have been suspended in 27.8 ml (200 mmoles) of triethylamine.

The suspension has been heated at 80°C and 18.9 ml (200 mmoles) of acetic anhydride have been added drop-wise.

At the end of the addition the solution has been kept at 80°C for 60' then it has been cooled at room temperature.

The reaction mixture has been diluted with 30 ml of toluene and extracted twice with 30 ml of a saturated solution of sodium bicarbonate.

After separation of the layers, the inorganic layer has been dried over sodium sulphate.

47 g (0.80 moles) of potassium hydroxide have been added to the dried solution and the suspension has been heated at 100 °C.

28 ml (0.22 moles) of 4-chlorobenzyl chloride have been added drop-wise over a period of about 90 minutes and the mixture has been then kept at reflux for further 60'.

After cooling up to room temperature the suspension has been diluted with 50 ml of water and left under stirring until the complete dissolution of the salts occurred. Layers were then settled and separated and the organic phase has been washed with 25 ml of 10% sodium chloride and dried over sodium sulphate The solution has been finally concentrated to a small volume and purified by means FCC on silica gel (8:2 toluene/ethyl acetate as eluent).

The fractions containing the product were pooled and concentrated to obtain 22.6 grams (73%) of methyl 2,3,4-tri-O-(4-chlorobenzyl)-6-deoxy-L-galactopyranoside (colourless syrup).

¹H NMR (200 MHz, CDCl3): 7.34-7.18 (m, 12H, H Ar), 4.91-4.57 (m, 6H, 3CH₂ benzyl), 4.25 (d, 7.6 Hz, 1 H, H1-β), 3.98 (dd, 1 H, J_{2,3} 1.1Hz, J_{2,1} 3.4Hz, H2-α), 3.88 (dd, 1H, J_{3,4} 2.7HZ, J_{3,2} 10.1Hz, H3-α), 3.85 (m, 1 H, H5-α), 3.61 (dd,1H, J_{4,5} 0.9Hz, J_{4,3} 2.4 Hz, H4-α), 3.54 (s, 3H, OCH₃-β), 3.37 (s, 3H, OCH₃-α), 1.24 (d, 6.4Hz, 3H, H6-β), 1.17 (s, 6.4Hz, 3H, H6-α).

¹³C NMR (50 MHz, CDCl₃), α Anomer: 98.6, 79.2, 78.6, 76.5, 74.4, 72.6, 72.6, 66.1, 55.4, 16.7. β Anomer: 104.9, 82.4, 79.4, 77.2, 74.2, 74.2, 72.4, 70.4, 57.0, 16.9.

### Example 5

### Preparation of crude Methyl 2,3,4-Tri-O-(4-Chlorobenzyl)-6-Deoxy-L-Galactopyranoside (4).

### (Compound of formula V with P= 4-chlorobenzyl)

100 g (0.56 moles) of the **3** have been treated as described in the example 4 excluding the purification step on silica.

After processing the reaction mixture and concentration of the organic layer a 387.4 g of the syrup have been obtained. The syrup was employed in the subsequent step without further purification.

### Example 6

### Preparation of 2,3,4-Tri-O-(4-Chlorobenzyl)-6-Deoxy-L-Galactopyranoside (5). (Compound of formula_VI with P= 4-chlorobenzyl)

A crude syrup of **4** (387.4 g, 0.41 moles), prepared as described in the example 5 has been treated with 1500 ml of 80% acetic acid and 280 ml of 2M Hydrochloric acid

After heating for 10 hours at reflux the solution has been cooled at room temperature and diluted with 1 liter of dichloromethane.

Then the layers were separated and the organic layer was first washed with a saturated solution of sodium bicarbonate and then with water After drying over sodium sulphate and concentration to syrup, the crude product was crystallized from hexane.

155.2 grams of **5** (289 mmoles, yield 70% from **4,** 52% from **3**) have been obtained.

¹H NMR (300 MHz, CDCl₃): 7.33-7.20 (m, 12H, H Ar), 5.28 (m, 1H, H1-α), 4.88 (m, 2H benzyl), 4.75-4.59 (m, 5H, 4H benzyl + H1-β), 4.11 (q, 6.6 Hz, 1H, H-5α), 3.97 (dd, 1H, J_{2,3} 10.4Hz, J_{2,1} 3.7 Hz, 1H, H2-α), 3.87 (dd, J_{3,2} 10.4Hz, J_{3,4} 2.7 Hz, 1H, H3-α), 3.70-3.46 (m, 5H, H2-β, H3-β, H4-β, H5-β, H4-α), 3.22 (bs, 1 H, 1OH-β), 2.90 (bs, 1H, 1OH-α), 1.24 (d, 6.6Hz, 3H, H6-β), 1.17 (d, 6.6Hz, 3H, H6-α).

### Example 7

### Preparation of 2,3,4-Tri-O-(4-Chlorobenzyl)-6-Deoxy-L-Galactopyranoside 1-O-Trichloroacetimidate (6).

### (Compound of formula IIa with P= 4-chlorobenzyl)

42 ml di trichloroacetonitrile and 1.1 ml (7.4 mmoles) of 1,8- diazabicyclo [5.4.0]undec-7-ene have been added to a solution of 5 (50.0 grams, 92 mmoles), in anhydrous dichloromethane (250 ml),

After 30' under stirring the reaction mixture has been extracted with 2x150 ml of saturated solution of ammonium chloride.

The organic layer has been dried oversodium sulphate , filtered and concentrated under vacuum.

The crude trichloroacetoimidate oil (73.8 grams) (6) so obtained has been employed without further purifications in the coupling reactions.

¹H NMR (300MHz, CDCl₃): 8.64 (s, 1 H, NH-β), 8.52 (s, 1 H, NH-α), 7.32-7.18 (m, 12H, H aromatics), 6.53 (d, 3.3 Hz, 1H, H1-α) 5.72 (d, 8.1 Hz, 1H, H1-β), 4.95-4.60 (m, 6H, 3CH₂ benzyl), 4.17 (dd, 1H, J_{2,3} 10.2 Hz, H2-α), 4.11 (q, 6.6 Hz, 1, H5-α), 4.01 (m, 1H, H2-β), 3.97 (dd, 1H, J_{3,4} 2.7 Hz, H3-α), 3.72-3.64 (m, 2H, H4-αβ + H5-β), 3.62-3.55 (m, 2H, H3-β + H4-β), 1.27 (d, 6.4 Hz, 3H, H6β), 1.20 (d, 6.3 Hz, 3H, H6α).

### Example 8

### Preparation of Methyl 2,3,4-Tri-O-(2-Chlorobenzyl)-6-Deoxy-L-Galactopyranoside (7). (Compound of formula V with P= 2-chlorobenzyl)

3.0 g (16.8 mmoles) of 3 have been treated as in the example 4, using 8.3 ml (60 mmoles) of triethylamine, 5,7 ml of acetic anhydride (60 mmoles), 14 g of KOH (0,24 moles) and 8.5 ml (67 mmoles) of 2-chlorobenzyl chloride.

At the end of the work-up, the solution has been concentrated up to a reduced volume under vacuum and purified on silica gel (hexane/ethyl acetate 8:2 as eluent). The fractions containing the product have been pooled and concentrated to give 6,4 grams (69%) of Methyl 2,3,4-Tri-O-(2-Chlorobenzyl)-6-Deoxy-L-Galactopyranoside (7) as a colourless syrup.

¹H NMR (300MHz,CDCl₃): 7.0-7.1 (m,12H, H Ar), 5.14-4.73 (m, 7H, 3CH₂ benzyls α and β, H1-α), 4.30 (d, 7.5Hz, H1-β), 4.13 (dd, 1H, J_{2,3} 2.6 Hz, J_{2,1} 3.3 Hz, H2-α), 4.06 (dd, 1 H, J_{3,4} 2.7 Hz, J_{3,2} 10.2 Hz, H3-α), 3.96 (q, 1 H, 6.6 Hz, H5-α), 3.83 (d, 1 H, 1.5 Hz, H4-α), 3.57 (s, 3H, OCH₃-α), 3.43 (s, 3H, OCH₃-β), 1.33 (d, 3H, 6.6 Hz, H6-β), 1.25 (d, 3H, 6.6 Hz, H6-α).

¹³C NMR (75 MHz, CDCl₃), α anomer: 137.0-126.6 (C Ar), 98.72 (C1), 79.64, 79.13, 76.92, 72.21, 70.47, 70.43, 66.32, 55.52 (OCH₃), 16.66(C6). β anomer: 137.0-126.6 (C Ar), 105.02 (C1), 82.86, 79.56, 78.14, 72.10, 71.79, 70.47, 70.33, 57.22 (OCH₃), 16.89 (C6).

### Example 9

### Preparation of crude Methyl 2,3,4-Tri-O-(2-Chlorobenzyl)-6-Deoxy-L-Galactopyranoside (7).

### (Compound of formula V with P= 2-chlorobenzyl)

10.0 g (56 mmoles) of **3** have been treated as described in the example 8 excluding the purification step on silica gel. After the processing of the reaction mixture and the concentration of the organic layer 40.8 g of syrup have been obtained that were used in the subsequent step without further purification.

### Example 10

### Preparation of 2,3,4-Tri-O-(2-Chlorobenzyl)-6-Deoxy-L-Galactopyranoside (8) (Compound of the formula VI with P= 2-chlorobenzyl)

150 ml of 80% acetic acid and 28 ml of 2M hydrochloric acid have been added to a crude syrup of 7 (40.8 g, 39 mmoles), prepared as described in the example 9. After heating for 10 hours at reflux the solution has been cooled at room temperature and diluted with 100 ml of dichloromethane. Layers were separated and the organic layer was first washed with a saturated solution of sodium bicarbonate and then with water.

After drying with sodium sulphate and concentration up to a syrup the crude product was crystallized from hexane.

14.9 grams (28 mmoles, 72% yield from **7,** 50% from **3**) have been obtained.

1 H NMR (200MHz, CDCl₃): 7.60-7.19 (m, 12H, H Ar), 5.40 (d, 1 H, 3.2 Hz, H1-α), 5.23-4.71 (m, 13H, 3CH₂ benzyls, H1-β), 4.21 (q, 1H, 6.6 Hz, H5-α), 4.11 (dd, 1H, J_{2,3} 9.8 Hz, J_{2,1} 3.2 Hz, H2-α), 4.00 (dd, 1H, J_{3,2} 9.8 Hz, J_{3,4} 2.6 Hz, H3-α), 3.88-3.58 (m, 5H, H2-β, H3-β, H4-β, H5-β, H4-α), 1.31 (d, 3H, 6.4 Hz, H6-β), 1.25 (d, 3H, 6.6 Hz, H6-α).

¹³C NMR (50 MHz, CDCl₃): 137.0-126.6 (C Ar α + β)
α anomer: 91.73, 79.24, 78.57, 77.05, 72.05, 70.60, 70.01, 66.83,16.67;
β anomer: 97.61, 82.83, 80.85, 77.78, 72.04, 71.83, 70.88, 70.05, 16.87.

### Example 11

### Preparation of 2,3,4-Tri-O-(Z-Chlorobenzyl)-6-Deoxy-L-Galactopyranoside 1-O-trichloroacetimidate (9)

### (Compound of formula IIa with P= 2-chlorobenzyl)

8.5 ml of trichloroacetonitrile and 0,22 ml (1.5 mmoles) of 1,8-diazabiciclo[5.4.0]undec-7-ene have been added to a solution of **8** (10.0 grams, 18.6 mmoles), in anhydrous dichloromethane.

After 30' under stirring the reaction mixture has been extracted with 2x30 ml of an Ammonium chloride-saturated solution.

The organic layer has been dried over sodium sulphate, filtered and concentrated under vacuum.

The crude trichloroacetimidate (9) so obtained has been used in the coupling reactions without further purification.

¹H NMR (200 MHz, CDCl₃): 8.68 (s, 1H, NH β anomer), 8.57 (s, 1H, NH α anomer), 7.65-7.15 (m, 12H, aromatic hydrogens α+ β), 6.66 (d, 3.4 Hz, 1H, H1-α), 5.85 (d, 7.8Hz, 1 H, H1-β), 4.35 (dd, J_{2,3} 9.8 Hz, 1 H, H2-α), 4.26-4.14 (m, 2H, H3-α + H5-α), 4.19 (dd, J_{2,3} 9.4 Hz, 1 H, H2-β), 3.91 (dd, J 2.6 e 1.0 Hz, 1 H, H4-α), 3.86-3.84 (m, 3H, H3-β + H4-β + H5-β), 1.36 (d, 6.4 Hz, 3H, 3H6-β), 1.29 (d, 6.6Hz, 3H, 3H6-α).

¹³C NMR (50 MHz, CDCl₃):
α anomer: 161.20 (C=NH), 130.0-126.0 (C Ar), 95.23 (C1), 78.75, 78.39, 76.14, 72.1, 70.10, 70.04, 69.62, 16.63 (C6).
β anomer: 161.62 (C=NH), 130.0-126.0 (C Ar), 98.77 (C1), 82.70, 78.39, 77.63, 72.14, 71.85, 71.71, 70.24, 16.74 (C6).

### Example 12

### Preparation of O-[2,3,4-Tri-O-(4-Chlorobenzyl)-6-Deoxy-α-L-Galactopyranosyl]-(1→2)-O-(6-O-Benzoyl-3,4-O-Isopropyliden-β-D-Galactopyranosyl)-(1→4)-2,3:5,6-Di-O-Isopropilidene-D-Glucose Dimethylacetal (10).

### (Compound of the formula IIIa with P=chlorobenzyl and R₃= benzoyl)

A syrup of crude 2 (prepared as described in the example 2 starting from 5.1 grams, 10.0 mmoles, of 1) has been dissolved in anhydrous dichloromethane (32 ml) and added with trimethylsilyl trifluoromethanesulphonate (23µl).

A solution prepared by dissolving a syrup of crude **6** (prepared as described in the example 7 starting from 10.0 g, 18.6 mmoles, of 5) into 40 ml of anhydrous dichloromethane has been added to the solution under stirring.

At the end of the addition the solution has been kept at room temperature for 30'. The TLC of the mixture shows a main spot of the expected product ((R_{f} =0.7 in 2:1 n-hexane/ethyl acetate), a small amount of the condensation product with β configuration, the spot of the non-reacted acceptor **(2)** and some degradation spots of the glycosyl donor.

After neutralization with triethylamine (23µl), the reaction mixture has been concentrated to a small volume and passed through a column of silica gel (2:1 n-hexane/ethyl acetate).

9.71 grams (8.6 mmoles, 86% yield calculated from the acceptor **2**) of **10** have been obtained as a colourless syrup.

¹H NMR (300MHz, CDCl₃): 8.05 (d, 7.2 Hz, 2H Bz), 7.57 (m, 1H Bz), 7.45 (m, 2H, Bz), 7.30-7.20 (m, 12H Ar Bn), 5,59 (d, 3.0 Hz, 1H, H1"), 4.90-4.45 (m, 10H, 3CH₂ benzyls, H1', H2, 2H6'), 4.34 (d, 5.7Hz, 1H, H1), 4.27 (m, 1H), 4.20-4.00 (m, 9H), 3.91 (dd, J 6.6 e 8.1 HZ, 1H), 3.73, (dd, J 6.3 e 8.1 Hz, 1 H) 3.63 (bs, 1 H, H4"), 3.37 (s, 3H, OCH₃), 3.35 (s, 3H, OCH₃), 1.50 (s, 3H), 1.43 (s, 3H), 1.37 (s, 6H), 1.32 (s, 3H), 1.15 (d, 6.2 Hz, 3H, H6").

¹³C NMR (75 MHz, CDCl₃) 166.19 (ArCOO); 137.72, 137.39, 137.25, 133.18, 133.11, 133.03 (C quat Ar) 130.0-128.1 (CH Ar), 110.32, 109.86, 108,49 (C quat isopropylydene); 105.08 (C1), 101.06 (C1'), 94.79 (C1"); 80.3, 78.84, 78.68, 77.65, 76.46, 75.13, 74.19, 73.84, 72.23, 71.65, 70.86, 66.28; 64.97, 63.67 (CH₂e CH₂'), 56.00, 53.03 (2 OCH₃); 27.83, 27.11, 26.84, 26.70, 26.43, 25.02 (CH₃ of the isopropylydene groups); 16.81 (C6").

### Example 13

### Preparation of O-[2,3,4-Tri-O-(4-Chlorobenzyl)-6-Deoxy-α-L-Galactopyranosyl]-(1→2)-O-(3,4-O-Isopropyliden-β-D-Galactopyranosyl)-(1→4)-2,3:5,6-Di-O-Isopropyliden-D-Glucose Dimethylacetale (11).

### (Compound of formula IIIb' with P=4-chlorobenzyl)

4.00 g (3.5 mmoles) of 10 have been dissolved in 40 ml of methanol and 0.39 ml (3.9 mmoles) of a 30% sodium hydroxide solution have been added.

The solution has been kept at room temperature overnight and then it has been concentrated under vacuum up to a syrup.

The residue has been dissolved into 20 ml of dichloromethane and washed twice with 20 ml of water.

After separation , the organic layer wasdried over sodium sulphate and concentrated under vacuum.

3.5 g of 11 (3.4 mmoles, 97%) have been obtained as a colorless syrup.

¹H NMR (200 MHz, CDCl₃): 7.3-7.2 (m, 12H Ar), 5.57 (d, 2.4 Hz, 1H, H1"), 4.92-4.52 (m, 8H, 3 CH2 benzyls + H2+ H1'), 4.35 (d, 6.8 Hz, 1H, H1), 4.30-3.50 (m, 15H), 3.48 (s, 6H, 2-OCH3), 1.46 (s, 3H), 1.43 (s,3H), 1.38 (s, 6H), 1.29 (s, 6H), 1.13 (d, 6.4 Hz, 3H, H6").

¹³C NMR (50 MHz, CDCl₃): 137.71, 137.39, 137.30, 133.27, 133.18, 132.93 (quat Ar), 130.0-128.0 (CH Ar), 110.54, 109.78, 108.71 (C quat isopropylidene), 107.60 (C1), 101.45 (C1'), 94.94 (C1"), 80.88, 78.86, 78.77, 78.04, 77.58, 76.49, 75.52, 75.07, 74.58, 74.25, 74.06, 72.29, 71.77, 66.29, 64.93 (C6), 62.46 (C6'), 57.72 (OCH₃), 54.14 (OCH₃), 16.90 (C6").

### Example 14

### Preparation of O-[2,3,4-Tri-O-(4-Chlorobenzyl)-6-Deoxy-α-L-Galactopyranosil]-(1→2)-O-(6-0-Benzoyl-β-D-GAlactopyranosil)-(1→4)-D-Glucose (12).

### (Compound of t formula IIIb" with P= 4-chlorobenzyl and R₃= benzoyl)

5.00 g (4.4 mmoles) of **10** have been dissolved in 40 ml of acetonitrile. The solution has been heated at 40°C, added of 7,5 ml of 2M hydrochloric acid and kept at 40°C for 40'.

After said period the TLC (2:1 hexane/ethyl acetate) showed the absence of the starting material.

The solution has been then cooled at room temperature, neutralized with sodium hydroxide and concentrated under,vacuum.

The residue has been dissolved into 30 ml of dichloromethane and the solution has been washed twice with 20 ml of water.

The water washings have been extracted with 10 ml of dichloromethane and the pooled organic layers have been dried over sodium sulphate.

After elimination of the solvent under vacuum, the crude product has been crystallized from isopropyl ether. 3.37 grams (3.4 mmoles, 77%), of **12** have been obtained.

Melting Point= 111-112 °C.

¹H NMR (200 MHz, CDCl₃+ D₂O): 7.98 (pst,7.0 Hz, 2H Ar), 7.49 (m, 1H Ar), ), 7.35 (m, 2H Ar), 7.35-7.20 (m, 12H Ar), 5.22 (d, 3.2 Hz, 1 H, H1"), 4.88-4.33 (m, 10H, 3 CH₂ benzyls, H1, H1', 2 H6'), 411-3.21 (m, 14H, H2, H3, H4, H5, 2 H6, H2', H3', H4', H5', H2", H3", H4", H5"), 1.17-1.11 (m, 3H, H6").

¹³C NMR (50 MHz, CDCl₃): 166.60 (C quat ArCOO), 136.99, 136.83, 135.64, 133.68, 133.56, 130.06, 129.65, 129.60, 129.37, 128.81, 128.57, 101.88 (C1' a + β), 99.94 (C1"-β), 99.83 (C1"-α), 96.48 (C1-β), 92.31 (C1-α), 79.63, 79.21, 78.46, 78.06, 77.49, 75.22, 75.07, 74.49, 73.85, 73.25, 72.77, 72.64, 72.19, 71.73, 70.52, 68.39, 68.30, 63.23, 63.02, 61.23, 61.06, 16.80 (C6").

### Example 15

### Preparation of O-[2,3,4-Tri-O-(4-Chlorobenzyl)-6-Deoxy-α-L-Galactopyranosyl]-(1→2)-O-β-D-Galactopyranosyl-(1→4)-D-Glucose (13) via intermediate IIIb'

### (Compound of formula IVa with P=chlorobenzyl and R₄= H)

The reaction described in example 12 has been performed again starting from 19.5 grams (31 mmoles) of 2 as a crude product and 6 grams (prepared starting from 25.0 g, 46 mmoles of 5).

After neutralization with triethylamine the reaction mixture has been concentrated under vacuum up to obtain 54.9 grams of a syrup.

The syrup has been dissolved in 250 ml of methanol and 3.6 ml (15.7 mmoles) of 30% sodium methoxide in methanol have been added to the solution.

The reaction has been kept at room temperature (for about 24 hours) up to the complete disappearance of the starting product **10** as shown by the TLC control (with 2:1 n-hexane/ethyl acetate).

After neutralization with acetic acid the solution has been concentrated under vacuum and the obtained residue has been dissolved into 200 ml of acetonitrile. The resulting solution has been heated at 40°C and 28 ml of 2 M hydrochloric acid have been added.

After a 45' time at 40°C the TLC control (with 1:1 ethyl acetate/exane) has shown the disappearance of the intermediate **11.**

The suspension has been than filtered on dicalite and neutralized up to pH 6.5-7.0 by adding 30% sodium hydroxide.

After slow cooling at room temperature a white crystal precipitate has been obtained.

The solid product has been then filtered, washed with water and dried under vacuum at 50°C.

15.6 grams (17.8 mmoles) of 13 have been obtained, yield 57% from 2.
[α]D²⁰=-84.7 (C=1.0 in MeOH, to the equilibrium ). P_{fus} = 118-124°C.
HPLC purity: 96%.

¹H NMR (300 MHz, DMSO-d6): 7.4-7.2 (m, 12H aromatics), 6.65 (d, 6.6 Hz, OH β anomer), 6.33 (d, 4.5 Hz, OH α anomer), 5.54 (dd, 2.4 Hz, 1H, H1"), 5.00-4.52 (m, 11.4 H, 5OH, 6 CH2 benzyls, H1-α), 4.39-4.12 (m, 3.6 H, 1 OH, H1', H1-β, H5"), 3.85-2.95 (m, 15H), 1.08, 1.96 (2 d overlapped, 6.0 Hz, 3H, H6").

¹³C NMR (75 MHz, DMSO-d6): 138.09, 138.06, 137.90, 131.88, 131.79, 131.77 (6C quat Ar); 129.46, 129.08, 129.06, 128.17, 128.10, 128.07 (CH aromatics); 101.03, 100.89 (C1' α e β); 96.59 (C1, β); 95.97 (C1" α + β); 91.96 (C1, α), 79.52, 78.88, 78.04, 77.96, 75.41, 75.25, 75.19, 74.83, 74.68, 74.51, 73.56, 73.46 (CH2 benzyl), 72.34, 70.94 (CH2 benzyl), 70.30, 69.44 (CH2 benzyl), 68.72, 65.39; 60.31, 60.15, 60.11 (C6 e C6' α e β); 16.32, 16.23 (C6" α e β).

### Example 16

### Preparation of O-[2,3,4-Tri-O-(4-Chlorobenzyl)-6-Deoxy-α-L-Galactopyranosyl]-(1→2)-O-β-D-Galactopyranosyl-(1→4)-D-Glucose (13) via intermediate IIIb".

### (Compound of formula IVa with P=chlorobenzyl and R₄= H)

The reaction described in the example 12 has been performed again starting from 19.5 grams (31 mmoles) of 2 as a crude product and 36.9 grams of 6 as a crude product (prepared starting from 25.0 g, 46 mmoles of the 5).

After neutralization with triethylamine the reaction mixture has been concentrated under vacuum up to obtain 54.2 grams of a syrup.

The syrup has been dissolved in 200 ml of acetonitrile and the solution has been heated at 40°C.

Then 28 ml of 2M chloride acid have been added and the reaction mixture has been kept at 40°C for a 40' time , then it was cooled at room temperature.

After positive (absence of the **10** starting product spot) TLC control (with 2:1 ethyl acetate/hexane), the mixture has been neutralized with sodium hydroxide and concentrated under vacuum.

The residue has been dissolved into 250 ml of dichloromethane and the solution has been washed twice with 100 ml of water.

The organic layer has been concentrated under vacuum and the syrup has been dissolved into 250 ml of methanol.

After addition of 3.6 ml (15.7 mmoles) of 30% sodium methoxide in methanol, the solution has been kept at room temperature for 24 hours.

The mixture has been neutralized with acetic acid, discoloured with charcoal, filtered on dicalite and concentrated again under vacuum.

After crystallization from isopropyl ether 13.3 g (15.2 mmoles) of **13,** have been obtained, 49% yield from **2**.

[α_{]D}²⁰=-83.9 (C=1.0 in MeOH, to the equilibrium). Melting Point= 114-124°C.

94% of HPLC purity. NMR data in accordance with those reported in example 15.

### Example 17

### Preparation of O-[2,3,4-Tri-O-(2-Chlorobenzyl)-6-Deoxy-α-L-Galactopyranosyl]-(1→2)-O-(6-O-Benzoyl-3,4-O-Isopropylidene-β-D-Galactopyranosyl)-(1→4)-2,3:5,6-Di-O-Isopropylidene-D-Glucose Dimetylacetal (14).

### (Compound of formula IIIa with P= 2-chlorobenzyl e R₃= benzoyl)

A crude syrupof **2** (prepared as described in the example 2 starting from 5.1 grams, (10.0 mmoles), of **1** has been dissolved in anhydrous dichloromethane (32 ml) and added with trimethylsilyl trifluoromethansulphonate (23µl).

A solution prepared by dissolving a crude syrup of **9** (prepared as described in the example 11 starting from 10.0 g, 18.6 mmoles,of **8)** in 40 ml of anhydrous dichloromethane, has been added drop-wise to the solution under stirring.

At the end of the addition the solution has been kept at room temperature for a 30' time.

The TLC performed on a sample of the mixture shows a main spot of the expected product (R_{f} =0.7 in hexane/ethyl acetate 2:1), the spot of the non-reacted acceptor (2) and some spots of the degraded glycosyl donor.

After neutralization with triethylamine (23µl), the reaction mixture has been concentrated to small volume and passed through a silica gel column (with hexane/ethyl acetate 2:1).

6.91 grams of **14** (6.1 mmoles, 61% yield from acceptor **2**) have been obtained as a colourless syrup.

¹H NMR (300MHz, CDCl₃): 8.05 (d, 7.2 Hz, 2H Ar), 7.65-7.14 (m, 15 H Ar), 5.15-4.70 (m, 7H, 3CH2 benzyls + H2), 4.56-4.48 (m, 3H, 2H6' + h1'), 4.36 (d, 5.7 Hz, 1 H, H1), 4.34-3.94 (m, 11H), 3.84 (m, 1 H), 3.78 (m, 1 H), 3.37 (s, 3H, OCH3), 3.35 (s, 3H, OCH3), 1.51 (s, 3H), 1.48 (s, 3H), 1.41 (s, 3H), 1.38 (s, 3H), 1.30 (s, 3H), 1.28 (s, 3H), 1.22 (d, 6.6 Hz, 3H, 3H6").

¹³C NMR (75 MHz, CDCl₃): 166.42 (ArCOO),137.5-126.5 (C Ar), 110.47, 110.12, 108.73 (C quat isopropylidene); 105.17(C1), 101.36 (C1'), 95.00 (C1"); 80.31, 79.61, 79.17, 77.87, 77.83, 77.00, 75.37, 75.23, 74.02, 73.91, 72.26, 71.04, 70.04, 69.65, 66.65, 65.08, 63.89; 56.14, 53.12 (2 OCH₃); 27.96, 27.30, 27.01, 26.77, 26.45, 24.95 (6 CH₃ isopropylidene); 16.90 (C6").

### Example 18

### Preparation of O-(2,3,4-Tri-O-(2-Chlorobenzyl)-6-Deoxy-α-L-Galactopyranosyl]-(1→2)-O-(3,4-O-Isopropylidene-β-D-Galactopyranosyl)-(1→4)-2,3:5,6-Di-O-Isopropylidene-D-Glucose Dimetylacetal (15).

### (Compound of the formula IIIb' with P=2-clorobenzyl)

2.20 g (1.9 mmoles) of 14 have been dissolved in 40 ml of methanol and 0.21 ml (2.1 mmoles) of a 30% sodium hydroxide solution have been added to the solution.

The solution has been kept at room temperature for 2.5 hours then it has been concentrated under vacuum up to syrup.

The residue has been dissolved in 15 ml of dichloromethane and washed twice with 2 10 ml of water.

After separation, the organic layer has been dried over sodium sulphate and concentrated under vacuum.

1.85 g (1.8 mmoles, 94%) of colourless syrup have been obtained.

¹H NMR (200 MHz, CDCl₃): 7.65-7.10 (m, 12H Ar), 5.68 (d, 3.4 Hz, 1H, H1"), 5.16-4.68 (m, 7H, 3 CH2 benzyls + H2), 4.61 (d, 8.0 Hz, 1 H, H1'), 4.36 (d, 6.6 Hz, 1 H, H1), 4.32-3.60 (m, 15H), 3.49 (s, 6H, 2 OCH3), 1.50 (s, 3H), 1.48 (s, 3H),1.39 (s, 6H), 1.29 (s, 3H), 1.27 (s, 3H), 1.20 (d, 6.6 Hz, 3H, 3H6").

¹³C NMR (50 MHz, CDCl₃): 137.07, 136.87, 136.79, 132.65, 132.61, 133.15, 129.74, 129.68, 129.04, 129.01, 128.63, 128.46, 128.34, 128.25, 126.3, 126.73, 126.63 (C Ar); 110.59, 109.92, 108.80 (C quat. Isopropylidenes); 107.66 (C1), 101.65 (C1'), 95.03 (C1"), 80.91, 79.65, 79.13, 78.25, 77.74, 76.99, 75.63, 75.08, 74.67, 74.29, 74.09, 72.27, 70.38, 69.57, 66.54, 64.93, 62.55, 57.80 (OCH3), 54.08 (OCH3); 28.02, 27.22, 26.84, 26.80, 26.48, 24.88 (CH3 isopropylidenes), 16.92 (C6").

### Example 19

### Preparation of O-[2,3,4-Tri-O-(2-Chlorobenzyl)-6-Deoxy-α-L-Galactopyranosyl]-(1→2)-O-β-D-Galactopyranosyl-(1→4)-D-Glucose (16) through the intermediate IIIb'

### (Compound of formula IVa with P= 2-chlorobenzyl and R₄= H)

The reaction described in example 17 has been performed again with the same modalities and quantities.

After neutralization with triethylamine the reaction mixture has been concentrated under vacuum to obtain 20.8 g of a syrup.

The syrup has been dissolved in 100 ml of methanol and 1.1 ml (19 mmoles) of 30% sodium hydroxide have been added to the solution.

The reaction has been kept at room temperature until the TLC control (n-hexane/ethyl acetate 2:1) has shown the complete absence of the starting product **14.** After neutralization with acetic acid the solution has been concentrated under vacuum and the residue has been dissolved in 80 ml of acetonitrile.

The resulting solution has been heated at 40°C and added with 11.2 ml of 2 M hydrochloric acid. After a 30' time at 40°C the TLC control has shown the absence of the intermediate product **15.** The suspension has been then filtered on dicalite and neutralized at pH 6.5-7.0 by adding 30% sodium hydroxide. A white and crystal precipitate has been obtained after slow cooling at room temperature which has been then filtered and washed with water.

4.65 g (5.3 moles, 53% yield from **2**) of **16** have been obtained after drying under vacuum.

[α]_{D}²⁰=- 65.7 (C=1.0 in MeOH, to the equilibrium), P_{fus} = 125-130°C

¹H NMR (300 MHz, DMSO-d6): 7.75-7.20 (m, 12H aromatics), 6.68 (d, 6.6 Hz, 0.7 H, OH β anomer ), 6.35 (d, 4.5 Hz, 0.3 H, OH α anomer ), 5.63 (bs, 1 H, H1"), 5.05-4.60 (m, 11,3 H, 3CH2 benzyls, 5 OH, H1-α), 4.45-4.23 (m, 2.7 H, H1', H5", H1-β), 4.26 (s, 0.7H, OH), 4.14 (s, 0.3H, OH), 4.00-3.25 (m, 14.3 H), 2.99 (m, 0.7H), 1.10 (pst, 6.0Hz, 3H, H6").

¹³C NMR (75 MHz, DMSO-d6): 136.45, 136.34, 136.29, 131.90, 131.63, 131.52 (6C quat Ar); 129.56, 129.42, 128.90, 127.04, 126.96, 126.89 (CH aromatics); 100.99, 100.83 (C1' α and β); 96.60 (C1, β); 96.04 (C1" α + β); 91.97 (C1, α), 79.41, 78.71, 78.56, 78.40, 76.00, 75.39, 75.21, 74.80, 74.70, 74.53, 73.69, 73.55, 72.36, 71.27 (CH2 benzyls), 70.98, 70.30, 69.50, 69.44 (CH2 benzyl), 68.66, 67.68 (CH2 benzyl), 65.47, 60.32, 60.15, 16.14 (C1"-α), 16.05 (C6"-β).

### Example 20

### Preparation of 2'-O-α-L-fucopyranosyllactose (17) from the intermediate 13

12.00 g (13.7 mmoles) of the 13 have been dissolved in 600 ml of methanol and 8.1 g (49.3 mmoles) of sodium acetate and 1.5 g of palladium 10% on charcoal have been added to the solution.

The reaction flask has been saturated with hydrogen and the suspension has been kept, under a vigorous stirring, at room temperature for 24 hours: The TLC control (Toluene,methanol,AcOH 10:10:1) shows that the reaction is complete.

The suspension has been then filtered on decalite, concentrated up to a syrup and deionised on a couple of ion exchange resins.

5.70 grams (85%) of 2'-α-L-fucopyranosyllactose have been obtained after concentration and crystallization from methanol/n-propanol.

HPLC purity: 99%

The NMR spectra is in agreement with the literature (Y. Ishizuka et al. J. Carbohyd. Chem. (1999), 18(5), 523-533).

### Example 21

### Preparation of 2'-O-α-L-fucopyranosyllactose (17) from the intermediate 16

4.00 g (4.6 mmoles) of the **16** have been dissolved in 200 ml of methanol and 2.7 g (16 mmoles) of sodium acetate and 0.5 g of palladium 10% on charcoal have been added to the solution.

The reaction flask has been saturated with hydrogen and the suspension has been kept, under a vigorous stirring, at room temperature for 24 hours. The TLC control (Toluene,methanol,AcOH 10:10:1) shows that the reaction is complete.

The suspension has been then filtered on decalite, concentrated up to a syrup and deionized on a couple of ion exchange resins.

1.98 grams (88%) of 2'-α-L-fucopyranosyllactose have been obtained after concentration and crystallization from methanol/n-propanol.

HPLC purity: 98%

The NMR spectra is in agreement with the literature (Y. Ishizuka et al. J. Carbohyd. Chem. (1999), 18(5), 523-533).

The following 22-26 examples report the experimental details of the synthesis as schematically showed as follows:

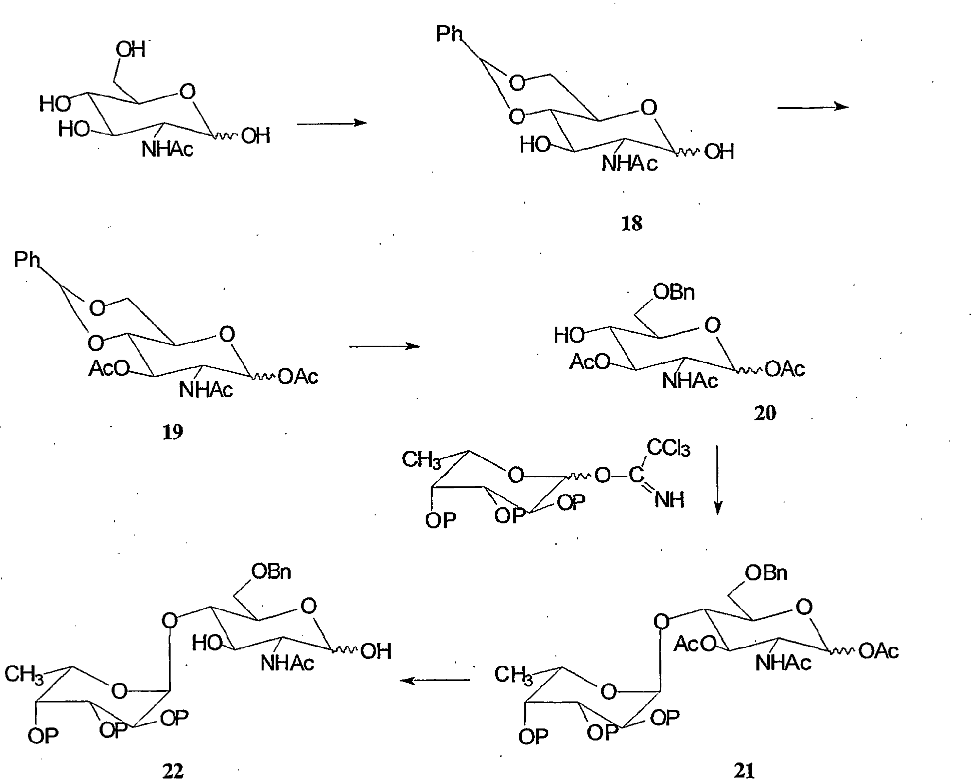

### Example 22

### Preparation of 4,6-O-Benzylidene-2-Acetamido-2-Deoxy-D-Glucopyranoside (18).

44.3 g (0.2 moles) of 2-acetamido-2-deoxy-D-glucopyranoside have been suspended in N,N-dimethylformamide and 3.82 g (20 mmoles) of p-toluen sulphonic acid and 50 ml of benzaldehyde dimethyl acetal have been added to the suspension.

The reaction has been heated uat 60° C and neutralized after 80 minutes with 3.5 ml (25 mmoles) of triethylamine.

The solution has been cooled at room temperature concentrated up to 150 g and crystallized with water. 44.2 g (71 %) have been obtained.

¹H (300 MHz, DMSO-d6) 7.81 (d, 8.1 Hz, 1H, NH), 7.49-7.37 (m, 5H, Ar), 6.75 (d, 5.7 Hz, 1H, OH 1), 5.61 (s, 1H, CHPh), 5.2 (d, 5.4 Hz, 1H, OH3-β), 5.05 (d, 5.4 Hz, 1H, OH3-α), 4.99 (t, J 4.2 e 3.6 Hz, 1H, H1-α), 4.62 (t, 1H, H1-β), 4.18 (dd, 6.0 Hz, 1 H, H6-β), 4.10 (dd, J 4.5 e 9.9 Hz, H6-α), 3.90-3.56 (m, 4H, H2, H4, H5, H6), 3.45 (t, 9.0 Hz, 1 H, H3), 1.85 (s, 3H, CH₃Ac-α), 1.83 (s, 3H, CH₃ Ac-β)

¹³C (75 MHz, DMSO-d6): 169.43 (COAc), 137.82 (C quat Ar), 128.81, 127.99, 126.35 (CH Ar), 100.85 (CH Ph-β), 100.67 (CH Ph-α), 96.0 (C1β), 91.45 (C1α), 82.46 (C4α), 81.51 (C4β), 70.49, 68.27, 67.94, 67.18, 65.85 (C6β), 62.05 (C6α), 58.03 (C2β), 54.74 (C2α), 23.07 (CH₃ Ac-β), 22.63 (CH₃ Ac-α)

### Example 23

### Preparation of 1,3-di-O-Acetyl-4,6-O-Benzylidene-2-Acetamido-2-Deoxy-D-Glucopyranoside (19).

20 grams (64.7 mmoles) of **18** have been suspended in acetonitrile (200 ml) and triethylamine (22.5 ml). 15 ml of acetic anhydride have been added drop-wise to the suspension, pre-heated at 50°C. At the end of the addition the solution has been kept at 65°C for some minutes until the formation of aprecipitate was observed.

The suspension has been cooled at room temperature and the solid product has been filtered and washed with acetonitrile.

12.1 g of the product have been obtained.

The mother liquors have been concentrated and further 11.8 g of the product have been recovered.

The total yield is 23.9 grams (94%).

¹H NMR (300 MHz, CDCl₃): 7.46-7.35 (m, 5H, Ar), 6.14 (d, J1,2 3:6 Hz, 1H, H1), 5.83 (bd, 1 H, NH), 5.54 (s, 1H, CHPh), 5.33 (t, 10.5 Hz, 1 H, H3), 4.48 (ddd, 1 H, H2), 4.30 (dd, 1 H, H6), 3.92 (dd, 1 H, H6), 3.79 (t, 9.6 Hz, 1 H, H5), 3.76 (t, 9.3, Hz, 1H, H4), 2.07, 1.94, 1.93 (3s, 9H, CH₃ Ac)

¹³C NMR (75 MHz, CDCl₃): 171.96, 170.24, 169.10, (COAc), 136.88 (C quat Ar), 129.36, 128.39, 126.27 (CH Ar), 101.79 (CH Ph), 91.35 (C1), 78.60, 69.95, 68.72 (C4, C3, C5), 65.04 (C6), 51.82 (C2), 23.11 (CH₃ NHAc), 22.63 (CH₃ OAc)

### Example 24

### Preparation of 1,3-di-O-Acetyl-6-O-Benzyl-2-Acetamido-2-Deoxy-D-Glucopyranoside (20)

5.0 grams (12.7 mmoles) of the 19 have been dissolved in tetrahydrofurane (100 ml) and kept under stirring at room temperature in an atmosphere of N₂, with ciano borohydride (4.0 g, 63.6 mmoles) and molecular sieves (10 g)

After an 1 hour 60 ml of 2% methanesulphonic acid in tetrahydrofurane have been added dropwise.

After a three hours time from the beginning of the addition, the solution has been cooled and neutralized with 75 ml of a solution saturated with hydrogen carbonate. The suspension has been filtered on dicalite and the aqueous layer has been extracted with dichloromethane.

The organic layers have been pooled and washed with a a saturated solution saturated sodium carbonate, then washed with water and dried over sodium sulphate.

The solution has been then concentrated to dryness and 4.1 g (80%) of the product have been obtained.

¹H NMR (300 MHz, CDCl₃): 7.36-7.31 (m, 5H, Ar), 6.13 (d, J_{1,2} 3.6 Hz, 1H, H1), 5.68 (d, J_{NH,2} 8.4, 1 H, NH), 5.12 (dd, J_{2,3} 11.1, J_{3,4} 8.7 Hz, 1 H, H3), 4.57 (q, 12 Hz, 2H, CH₂Bn), 4.34 (ddd, J 3.6, 8.7, 11.1, 1H, H2), 3.90-3.64 (m, 4H, H4, H5, 2H6), 3.04 (bs, 1H, OH4), 2.16, 2.12 (2s, 6H, CH₃ OAc), 1.87 (s, 3H, CH₃ NHAc)

¹³C NMR (75 MHz, CDCl₃): 172.49, 170.34, 169.26 (COAc), 137.45 (C quat Ar), 128.70, 128.19, 127.99 (CH Ar), 91.10 (C1), 74.10 (CH₂Bn), 73.07, 71.83, 70.22 (C3, C4, C5), 68.11 (C6), 51.25 (C2), 23.21 (CH₃ NHAc), 21.14 (2CH₃ OAc)

### Example 25

### Preparation of O-[2,3,4-Tri-O-(4-Chlorobenzyl)-6-Deoxy-α-L-Galactopyranosyl]-(1→4)-O-1,3-di-O-Acetyl-6-O-Benzyl-2-Acetamido-2-Deoxy-D-Glucopyranoside (21)

### (Compound of formula III with P=4-chlorobenzyl)

2.6 g (6.55 mmoles) of 20 have been treated as described in example 12. 2.95 g of a white solid (51%) have been obtained by using toluene/methanol=4/1 as eluent for the silica gel chromatography.

¹H NMR (300 MHz, CDCl₃): 7.29-7.14 (m, 15H Ar), 6.17 (d, 3.6 Hz, 1H, H1), 5.58 (d, 9.0 Hz, 1 H, NH), 5.19 (dd, 1H, H3), 5.00 (d, 3.6 Hz, 1 H, H1'), 4.83 (d, 11.4 Hz, 1 H, benzylic H) 4.65-4.40 (m, 7H, benzylic CH₂), 4.39-4.31 (m, 1H, H2), 3.95-3.74 (m, 7H, H4, H5, 2H6, H2', H3', H5'), 3.6 (bs, 1H, H4'), 2.17, 2.06 (2s, 6H, 2CH₃ OAc), 1.92 (s, 3H, CH₃ NAc), 1.12 (d, 6.3 Hz, 3H, H6').

¹³C NMR (75 MHz, CDCl₃): 172.44, 170.7, 169.00 (3 CO Ac), 138.30, 136.96, 136.90, 133.68, 133.61 (7C quat Ar); 129.57, 129.38, 128.70, 128.63, 128.51, 128.43, 128.32, 127.63, 127.59, 127.55 (CH Ar); 99.56 (C1'-α); 90.91 (C1-α), 79.01, 78.18, 76.38, 75.62, 74.43, 73.65, 73.10, 72.77, 72.09, 68.26 (C6), 67.55, 51.51 (C2), 23.12 (CH₃ NAc), 21.31, 21.12 (2 CH₃ AcO) 16.54 (C6').

### Example 26

### Preparation of O-[2,3,4-Tri-O-(4-Chlorobenzyl)-6-Deoxy-α-L-Galactopyranosyl]-(1→4)-O-6-O-Benzyl-2-Acetamido-2-Deoxy-D-Glucopyranoside (22)

### (Compound of formula IV with P= 4-chlorobenzyl)

1.25 g (1.37 mmoles) of **21** have been suspended in 7.5 ml of methanol and treated with 79 µl of 30 % sodium methoxide in methanol.

The reaction has been kept at room temperature for one hour.

The resulting suspension has been neutralized with acetic acid (23 µl) and treated with water (8 ml).

The solid product has been filtered and washed with methanol/water 1:1.

1.05 g of the product have been obtained.

¹H NMR (300 MHz, DMSO-d6): 7.71 (d, 7.8 Hz, NH), 7.4-7.2 (m, 17H Ar), 6.56 (d, 4.2 Hz, anomeric OH), 5.0 (d, 3.3 Hz, 1H, H1'-α), 4.96-4.94 (m, 1H, H1-α), 4.80-4.53 (m, 8H, OH, 3 CH₂Bn, H3), 4.35-4.26 (m, 3H), 3.85-2.95 (m, 10H), 1.84 (s, 3H, CH₃ NAc), (d, 6.6 Hz, 3H, H6').

¹³C NMR (75 MHz, DMSO-d6): 169.37 (CO Ac), 138.41, 138.02, 137.81, 137.60, 131.93, 131.87, 131.83 (7C quat Ar); 129.35, 129.32, 128.91, 128.17, 128.14, 128.11, 128.07, 127.33 (CH Ar); 97.46 (C1'α); 90.32 (C1α), 78.36, 77.89, 75.66, 73.45 (benzylic CH₂), 72.22, 71.98, 71.70, 70.34, 69.44 (benzylic CH₂), 69.23, 68.79, 66.29 (C6); 54.42 (C2), 16.35 (C6').

## Claims

1. Compounds 2,3,4-tri-O-benzyl-L-fucosyl derivatives of formula (IV) 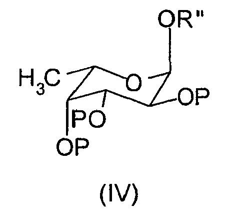 wherein
P independently among each other are benzyl groups of formula 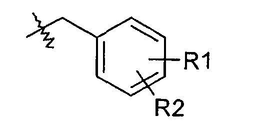 wherein R1 is chosen among chlorine, bromine, alkoxy and nitro; R2 is chosen among hydrogen, chlorine, bromine, alkoxy and nitro;
R" is a monosaccharide, disaccharide or oligosaccharide, whose hydroxy groups are free or partially protected by benzyl groups; wherein said hydroxy groups protected by benzyl groups are groups -OCH₂Ph or -OP; and wherein hydroxy groups partially protected by benzyl groups means that given a number n of hydroxyls present on R" at the most n/2 when n is an even number or (n-1)/2 when n is an odd number they are protected by said benzyl groups.

2. Compounds of formula (IV) according to claim 1 wherein R" is chosen among lactose, fucose, (2-acetylamino)-lactose, (2-amino)-lactose, (2-azido)-lactose, Lacto-N-biose, galactose, glucose, (2-acetylamino)-glucose, (2-amino)-glucose and (2-azido)-glucose whose hydroxy groups are free or partially protected by benzyl groups.

3. Compounds according to claim 2 wherein R" is chosen among 2'-lactose, 3- lactose, 3-(2-acetylamino)-lactose, 3-(2-amino)-lactose, 3-(2-azido)-lactose, 4-Lacto-N-biose, 2-galactose, 3-glucose, 3-(2-acetylamino)-glucose, 3-(2-amino)-glucose, 3-(2-azido)-glucose 4-(2-acetylamino)-glucose, 4-(2-amino)-glucose and 4-(2-azido)-glucose whose hydroxy groups are free or partially protected by benzyl groups.

4. Compounds according to claim 3 wherein R" is 2'-lactose that is compounds of formula (IVa) 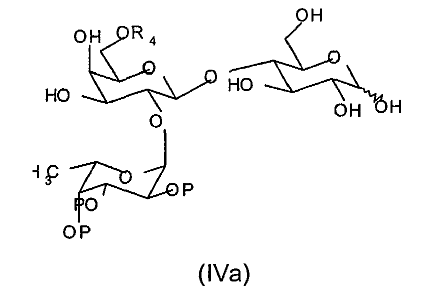 wherein P is as above described; R4 is hydrogen, benzyl or P.

5. A process for the synthesis of fucosyl derivatives of formula (I) 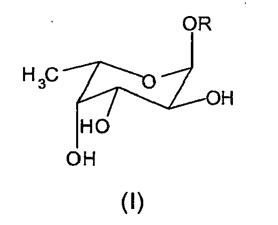 wherein
R is a monosaccharide, a disaccharide or an oligosaccharide with free hydroxy groups;
**characterised in that** it comprises the use of an intermediate of formula (IV) 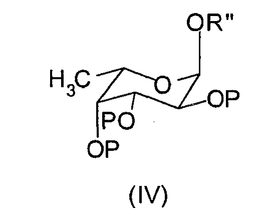 according to any one of claims 1-4.

6. Process according to claim 5 comprising a step c) of benzyl groups removal from intermediate (IV) to obtain a compound of formula (I).

7. Process according to claim 6 wherein step c) is preceded by the following steps:
*a) coupling* of a 2,3,4-tri-O-benzyl-fucopyranosyl derivative donor of formula (II) 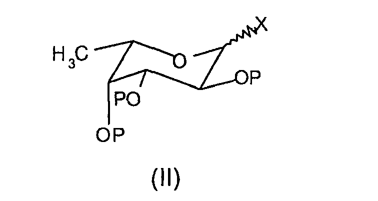 wherein P is as above described; X is an anomeric carbon activator;
with a monosaccaride, disaccaride or oligosaccaride glycosyl acceptor of formula R'OH wherein R' is, correspondingly to R, a monosaccharide, disaccharide or oligosaccharide suitably protected whose hydroxy groups for a maximum of 2 are free, optionally are partially protected by benzyl groups, the remaining hydroxy groups are protected with state of the art groups such to be removed in conditions such as to preserve benzyl groups and particularly to preserve groups -OP present on the fucosyl moiety;
to obtain intermediate of formula (III) 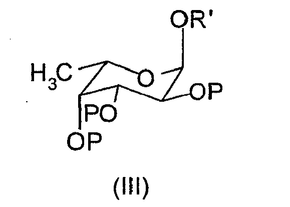 wherein P and R' are as above described;
b) removal of protecting groups of the saccharide moiety R' in conditions such as to preserve groups -OP present on the fucosyl moiety and optionally preserve benzyl groups present on R';
to obtain intermediates of formula (IV) 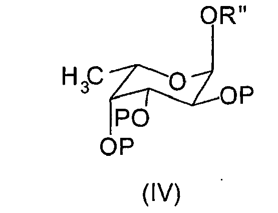 wherein R" and P are as above.

8. Compounds 2,3,4-tri-O-benzyl-L-fucosyl derivatives of formula (II) 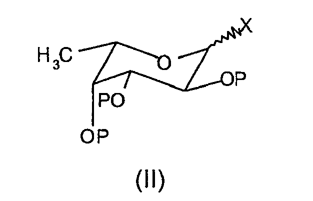 wherein
P independently among each other are benzyl groups of formula 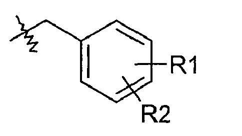 wherein R1 is chosen among chlorine, bromine, alkoxy and nitro; R2 is chosen among hydrogen, chlorine, bromine, alkoxy and nitro;
X is imidate;
excluding that for a compound of formula (II) R2 is hydrogen when X is trichloroacetimidate and R1 is p-chloro.

9. Use of compounds of formula (II) wherein
X is an anomeric carbon activator chosen among an imidate or an halogen;
P independently among each other are benzyl groups of formula 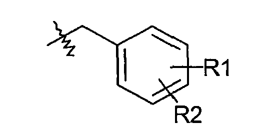 wherein R1 is chosen among chlorine, bromine, alkoxy and nitro; R2 is chosen among hydrogen, chlorine, bromine, alkoxy and nitro;
as fucosyl-donors for the synthesis of compounds of formula (IV) as described in claims 1-4.

10. Use according to claim 9 of compounds of formula (II) wherein X is trichloroacetimidate or α-bromine.

11. Use of compounds of formula (II) according to claims 9-10 as fucosyl-donors for the synthesis of compounds of formula (I) by means of the process according to claims 5-7.

## Patentansprüche

1. Verbindungen 2,3,4-Tri-O-benzyl-L-fucosylderivate der Formel (IV) 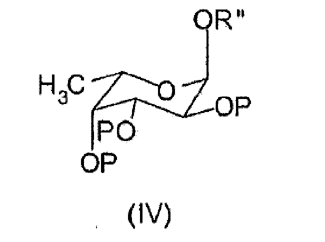 wobei
die Gruppen P unabhängig voneinander für Benzylgruppen der Formel 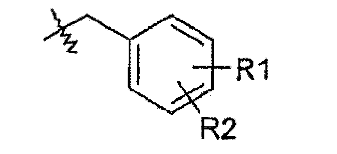 stehen, wobei R1 aus Chlor, Brom, Alkoxy und Nitro ausgewählt ist; R2 aus Wasserstoff, Chlor, Brom, Alkoxy und Nitro ausgewählt ist;
R" für ein Monosaccharid, Disaccharid oder Oligosaccharid steht, dessen Hydroxygruppen frei oder teilweise durch Benzylgruppen geschützt sind; wobei es sich bei den durch Benzylgruppen geschützten Hydroxygruppen um -OCH₂Ph- oder -OP-Gruppen handelt; und wobei teilweise durch Benzylgruppen geschützte Hydroxygruppen bedeutet, dass bei Vorliegen einer Zahl n von Hydroxygruppen an R" höchstens n/2, wenn n für eine gerade Zahl steht, oder (n-1)/2, wenn n für eine ungerade Zahl steht, der Hydroxygruppen durch die Benzylgruppen geschützt sind.

2. Verbindungen der Formel (IV) nach Anspruch 1, wobei R" aus Lactose, Fucose, (2-Acetylamino)-lactose, (2-Amino)lactose, (2-Azido)lactose, Lacto-N-biose, Galactose, Glucose, (2-Acetyl-amino)glucose, (2-Amino)glucose und (2-Azido)glucose, deren Hydroxygruppen frei oder teilweise durch Benzylgruppen geschützt sind, ausgewählt ist.

3. Verbindungen nach Anspruch 2, wobei R'' aus 2'-Lactose, 3-Lactose, 3-(2-Acetylamino)lactose, 3-(2-Amino)lactose, 3-(2-Azido)lactose, 4-Lacto-N-biose, 2-Galactose, 3-Glucose, 3-(2-Acetylamino)-glucose, 3-(2-Amino)glucose, 3-(2-Azido)glucose, 4-(2-Acetylamino)glucose, 4-(2-Amino)glucose und 4-(2-Azido)glucose, deren Hydroxygruppen frei oder teilweise durch Benzylgruppen geschützt sind, ausgewählt ist.

4. Verbindungen nach Anspruch 3, wobei R" für 2'-Lactose steht, d.h. Verbindungen der Formel (IVa) 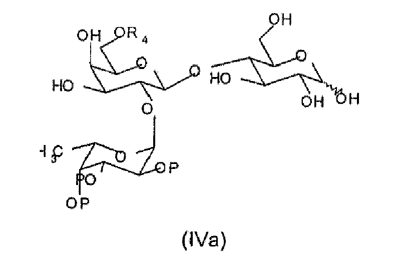 worin P die oben angegebene Bedeutung besitzt; R4 für Wasserstoff, Benzyl oder P steht.

5. Verfahren zur Synthese von Fucosylderivaten der Formel (I) 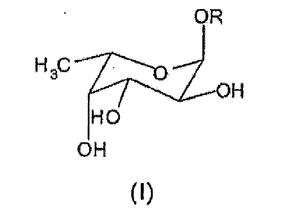 wobei
R für ein Monosaccharid, Disaccharid oder Oligosaccharid mit freien Hydroxygruppen steht;
**dadurch gekennzeichnet, dass** man ein Zwischenprodukt der Formel (IV) 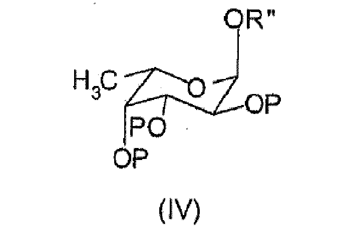 nach einem der Ansprüche 1-4 verwendet.

6. Verfahren nach Anspruch 5, umfassend einen Schritt c) der Benzylgruppenabspaltung von Zwischenprodukt (IV) zum Erhalt einer Verbindung der Formel (I).

7. Verfahren nach Anspruch 6, bei dem Schritt c) die folgenden Schritte vorgeschaltet werden:
a) *Kupplung* eines 2,3,4-Tri-O-benzyl-fuco-pyranosylderivat-Donors der Formel (II) 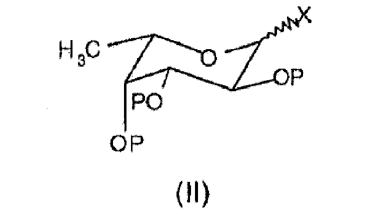 wobei P die oben angegebene Bedeutung besitzt; X für einen Aktivator des anomeren Kohlenstoffs steht;
mit einem Monosaccharid-, Disaccharid- oder Oligo-saccharidglycosyl-Akzeptor der Formel R'OH, wobei R', entsprechend R, für ein geeignet geschütztes Monosaccharid, Disaccharid oder Oligosaccharid steht, von dessen Hydroxygruppen höchstens 2 frei sind, gegebenenfalls teilweise durch Benzylgruppen geschützt sind, wobei die übrigen Hydroxygruppen so durch dem Stand der Technik entsprechende Gruppen geschützt sind, dass sie unter Bedingungen, unter denen Benzylgruppen erhalten bleiben und unter denen insbesondere an der Fucosylgruppierung vorliegende -OP-Gruppen erhalten bleiben, abgespalten werden;
zum Erhalt von Zwischenprodukten der Formel (III) 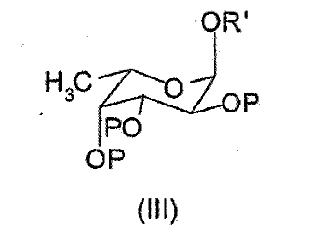 wobei P und R' die oben angegebene Bedeutung besitzen;
b) Abspaltung von Schutzgruppen von der Saccharidgruppierung R' unter Bedingungen, unter denen an der Fucosylgruppierung vorliegende -OP-Gruppen erhalten bleiben und gegebenenfalls an R' vorliegende Benzylgruppen erhalten bleiben;
zum Erhalt von Zwischenprodukten der Formel (IV) 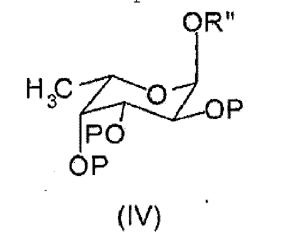 wobei R'' und P die oben angegebene Bedeutung besitzen.

8. Verbindungen 2,3,4-Tri-O-benzyl-L-fucosylderivate der Formel (II) 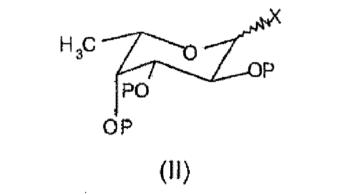 wobei
die Gruppen P unabhängig voneinander für Benzylgruppen der Formel 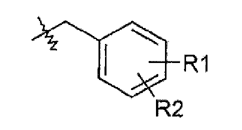 stehen, wobei R1 aus Chlor, Brom, Alkoxy und Nitro ausgewählt ist; R2 aus Wasserstoff, Chlor, Brom, Alkoxy und Nitro ausgewählt ist;
X für Imidat steht;
wobei ausgeschlossen ist, dass für eine Verbindung der Formel (II) R2 für Wasserstoff steht, wenn X für Trichloracetimidat steht und R1 für p-Chlor steht.

9. Verwendung von Verbindungen der Formel (II), wobei X für einen Aktivator des anomeren Kohlenstoffs, der unter einem Imidat oder einem Halogen ausgewählt ist, steht;
die Gruppen P unabhängig voneinander für Benzylgruppen der Formel 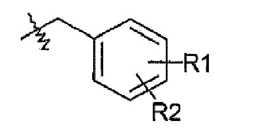 stehen, wobei R1 aus Chlor, Brom, Alkoxy und Nitro ausgewählt ist; R2 aus Wasserstoff, Chlor, Brom, Alkoxy und Nitro ausgewählt ist;
als Fucosyl-Donoren für die Synthese von Verbindungen der Formel (IV) gemäß den Ansprüchen 1-4.

10. Verwendung nach Anspruch 9 von Verbindungen der Formel (II), wobei X für Trichlorimidat oder α-Brom steht.

11. Verwendung von Verbindungen der Formel (II) nach den Ansprüchen 9-10 als Fucosyl-Donoren für die Synthese von Verbindungen der Formel (I) mit Hilfe des Verfahrens nach den Ansprüchen 5-7.

## Revendications

1. Composés dérivés de 2,3,4-tri-O-benzyl-L-fucosyle de formule (IV) 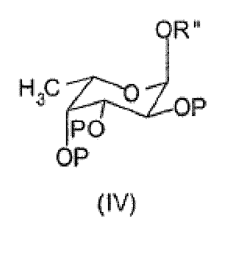 dans laquelle
P indépendamment les uns des autres sont des groupes benzyle de formule 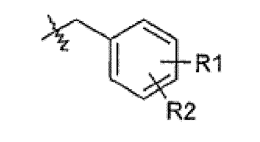 dans laquelle R1 est choisi parmi chlore, brome, alcoxy et nitro ; R2 est choisi parmi hydrogène, chlore, brome, alcoxy et nitro ;
R" est un monosaccharide, disaccharide ou oligosaccharide, dont les groupes hydroxy sont libres ou partiellement protégés par des groupes benzyle ; dans laquelle lesdits groupes hydroxy protégés par des groupes benzyle sont des groupes -OCH₂Ph ou -OP ; et dans laquelle les groupes hydroxy partiellement protégés par les groupes benzyle signifient qu'étant donné un nombre n d'hydroxyles présents sur R", au plus n/2 lorsque n est un nombre pair ou (n-1)/2 lorsque n est un nombre impair sont protégés par lesdits groupes benzyle.

2. Composés de formule (IV) selon la revendication 1, dans lesquels R" est choisi parmi lactose, fucose, (2-acétylamino)-lactose, (2-amino)-lactose, (2-azido)-lactose, Lacto-N-biose, galactose, glucose, (2-acétylamino)-glucose, (2-amino)-glucose et (2-azido)-glucose dont les groupes hydroxy sont libres ou partiellement protégés par des groupes benzyle.

3. Composés selon la revendication 2, dans lesquels R" est choisi parmi 2'-lactose, 3-lactose, 3-(2-acétylamino)-lactose, 3-(2-amino)-lactose, 3-(2-azido)-lactose, 4-Lacto-N-biose, 2-galactose, 3-glucose, 3-(2-acétylamino)-glucose, 3-(2-amino)-glucose, 3-(2-azido)-glucose, 4-(2-acétylamino)-glucose, 4-(2-amino)-glucose et 4-(2-azido)-glucose dont les groupes hydroxy sont libres ou partiellement protégés par des groupes benzyle.

4. Composés selon la revendication 3, dans lesquels R" est 2'-lactose, c'est-à-dire des composés de formule (IVa) 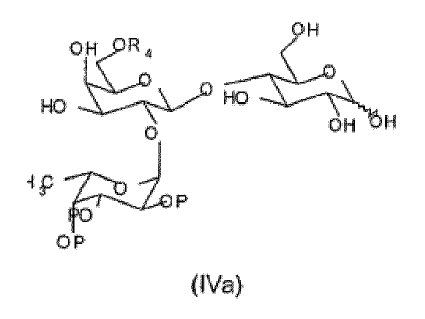 dans laquelle P est tel que décrit ci-dessus ; R4 est hydrogène, benzyle ou P.

5. Procédé de synthèse de dérivés de fucosyle de formule (I) 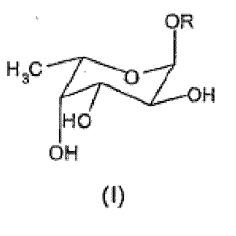 dans laquelle
R est un monosaccharide, un disaccharide ou un oligosaccharide avec des groupes hydroxy libres ;
**caractérisé en ce qu'**il comprend l'utilisation d'un intermédiaire de formule (IV) 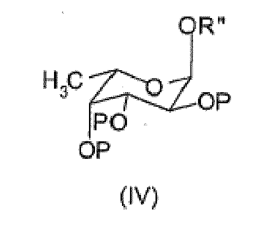 selon l'une quelconque des revendications 1 à 4.

6. Procédé selon la revendication 5, comprenant une étape c) d'élimination de groupes benzyle de l'intermédiaire (IV) afin d'obtenir un composé de formule (I).

7. Procédé selon la revendication 6, dans lequel l'étape c) est précédée par les étapes suivantes :
a) le couplage d'un donneur dérivé de 2,3,4-tri-O-benzyl-fucopyranosyle de formule (II) 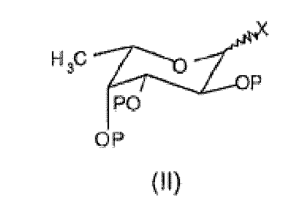 dans laquelle P est tel que décrit ci-dessus ; X est un activateur de carbone anomérique ;
avec un accepteur glycosyle de monosaccharide, disaccharide ou oligosaccharide de formule R'OH dans laquelle R' est, en correspondance avec R, un monosaccharide, disaccharide ou oligosaccharide protégé de façon appropriée dont les groupes hydroxy au maximum de 2 sont libres, facultativement sont partiellement protégés par des groupes benzyle, les groupes hydroxy restants sont protégés par des groupes de l'état de l'art de façon à être éliminés dans des conditions de façon à préserver des groupes benzyle et particulièrement à préserver des groupes -OP présents sur le fragment fucosyle ;
afin d'obtenir un intermédiaire de formule (III) 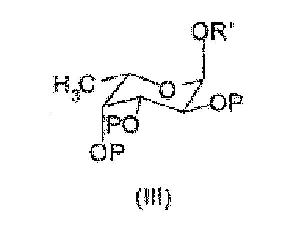 dans laquelle P et R' sont tels que décrits ci-dessus ;
b) l'élimination de groupes protecteurs du fragment saccharide R' dans des conditions de façon à préserver des groupes -OP présents sur le fragment fucosyle et facultativement préserver des groupes benzyle présents sur R' ;
afin d'obtenir des intermédiaires de formule (IV) 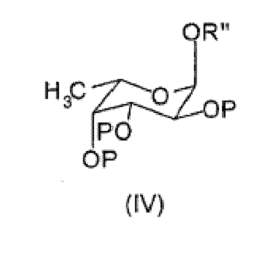 dans laquelle R" et P sont tels que ci-dessus.

8. Composés dérivés de 2,3,4-tri-O-benzyl-L-fucosyle de formule (II) 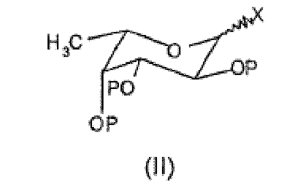 dans laquelle
P indépendamment les uns des autres sont des groupes benzyle de formule 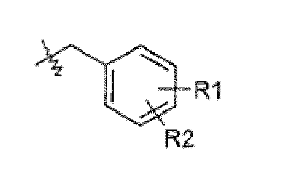 dans laquelle R1 est choisi parmi chlore, brome, alcoxy et nitro ; R2 est choisi parmi hydrogène, chlore, brome, alcoxy et nitro ;
X est imidate ;
à l'exclusion que pour un composé de formule (II), R2 soit hydrogène lorsque X est trichloroacétimidate et R1 est p-chloro.

9. Utilisation de composés de formule (II) dans laquelle
X est un activateur de carbone anomérique choisi parmi un imidate ou un halogène ;
P indépendamment les uns des autres sont des groups benzyle de formule 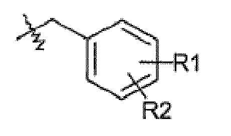 dans laquelle R1 est choisi parmi chlore, brome, alcoxy et nitro ; R2 est choisi parmi hydrogène, chlore, brome, alcoxy et nitro ;
en tant que donneurs de fucosyle pour la synthèse de composés de formule (IV) tel que décrit dans les revendications 1 à 4.

10. Utilisation selon la revendication 9 de composés de formule (II) dans laquelle X est trichloroacétimidate ou α-brome.

11. Utilisation de composés de formule (II) selon les revendications 9 et 10 en tant que donneurs de fucosyle pour la synthèse de composés de formule (I) au moyen du procédé selon les revendications 5 à 7.
